# FASCICULE DE BREVET EUROPEEN

(11) **EP 2 191 816 B1**
(45) Date de publication et mention de la délivrance du brevet: **18.09.2013**
(21) Numéro de dépôt: 09177091.7
(22) Date de dépôt: 25.11.2009
(51) Int. Cl.: A61K 8/49, A61Q 19/02

(54) **Procédé de dépigmentation des matières kératiniques à l'aide de composés dithiolanes.**
Aufhellung von keratinischem Material mit spezifischen Dithiolanen
Depigmentation of keratinic material using specific dithiolanes

(30) Priorité: 28.11.2008 FR 0858075
(43) Date de publication de la demande: 02.06.2010
(73) Titulaire: L'Oréal, 75008 Paris (FR)
(72) Inventeur: Marat, Xavier, 75010, Paris (FR); Lucet-Levannier, Karine, 92500 Rueil Malmaison (FR); Marrot, Laurent, 93190 Livry Gargan (FR)
(74) Mandataire: Kromer, Christophe

(56) Documents cités:
- EP-A- 0 308 919
- KUROBE HIROSHI ET AL: "Preparation and formulation of cyclic dithio derivatives as remedies for diabetic kidney diseases, hypoglycemic agents, hypolipidemic agents, and lenitives for digestive disorders" CAPLUS,, 4 juin 1998 (1998-06-04), XP002524712

## Description

La présente invention concerne un procédé de traitement cosmétique notamment pour dépigmenter et/ou blanchir la peau, employant au moins un composé de type dithiolane.

La couleur de la peau humaine est fonction de différents facteurs et notamment des saisons de l'année, de la race et du sexe; elle est principalement déterminée par la nature et la concentration de mélanine produite par les mélanocytes. Les mélanocytes sont les cellules spécialisées qui, par l'intermédiaire d'organelles particuliers, les mélanosomes, synthétisent la mélanine. A différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

L'utilisation de substances dépigmentantes topiques inoffensives présentant une bonne efficacité est toute particulièrement recherchée en vue de traiter les taches pigmentaires.

Le mécanisme de formation de la pigmentation de la peau, c'est-à-dire de la formation de la mélanine est particulièrement complexe et fait intervenir schématiquement les principales étapes suivantes :

Tyrosine ---> Dopa ---> Dopaquinone ---> Dopachrome ---> Mélanine

La tyrosinase (monophénol dihydroxyl phénylalanine: oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.
Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.
Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé est instable en solution, ce qui complique quelque peu la fabrication de la composition.

Il subsiste le besoin d'un nouvel agent blanchissant de la peau humaine à action aussi efficace que ceux connus, mais n'ayant pas leurs inconvénients, c'est-à-dire qui soit non irritant, non toxique et/ou non allergisant pour la peau, tout en étant stable dans une composition, ou bien alternativement qui possède une action renforcée de façon à pouvoir être utilisé en quantité plus faible, ce qui diminue considérablement les effets secondaires observés.
A cet égard, la Demanderesse a de manière surprenante et inattendue découvert que certains composés dithiolanes présentaient une bonne activité dépigmentante, même à faible concentration.

L'invention a donc pour objet un procédé cosmétique non thérapeutique de dépigmentation, d'éclaircissement et/ou de blanchiment des matières kératiniques, comprenant l'application d'une composition cosmétique comprenant, dans un milieu physiologiquement acceptable, au moins un composé de formule (I) telle que définie ci-après.
L'invention concerne également l'utilisation cosmétique non thérapeutique d'un composé de formule (I) comme agent blanchissant, éclaircissant et/ou dépigmentant des matières kératiniques.

Les composés utilisés selon l'invention permettent de dépigmenter et/ou d'éclaircir efficacement, voire de blanchir, la peau d'êtres humains. Ils sont notamment destinés à être appliqués sur la peau d'individus présentant des taches de pigmentation brunâtres, des taches de sénescence, ou sur la peau d'individus désirant combattre l'apparition d'une couleur brunâtre provenant de la mélanogénèse.
Ils peuvent également permettre de dépigmenter et/ou d'éclaircir les poils, les cils, les cheveux, ainsi que les lèvres et/ou les ongles.

Les composés utilisés selon l'invention répondent donc à la formule (I) suivante 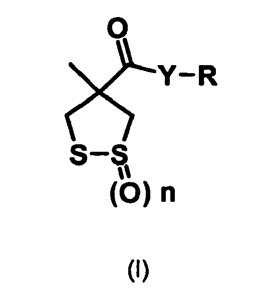 dans laquelle :
Y désigne O, NR₁ ou S
R₁ désigne un atome d'hydrogène ; un groupe hydrocarboné alkyle saturé linéaire en C₁-C₂₀ ou ramifié en C₃-C₂₀ ou insaturé en C₂-C₂₀ ; un groupe phényle éventuellement substitué par un ou plusieurs hydroxyles et/ou par un ou plusieurs radicaux alkoxy en C₁-C₈ ;
R désigne un atome d'hydrogène ; ou un groupe hydrocarboné alkyle saturé linéaire en C₁-C₂₀ ou ramifié en C₃-C₂₀ ou insaturé en C₂-C₂₀ ; ou un groupe phényle éventuellement substitué par un ou plusieurs hydroxyles et/ou par un ou plusieurs radicaux alkoxy en C₁-C₈ ; ou un groupe alkyle saturé en C₁-C₈ possédant un substituant phényle éventuellement substitué par un ou plusieurs hydroxyles et/ou par un ou plusieurs radicaux alcoxy en C₁-C8 ;.
R possède éventuellement un ou plusieurs substituants choisis parmi OR₂, SR₂, NR₂R₃, COOR₂ dans lesquels :
R₂ désigne un atome d'hydrogène ou un groupe hydrocarboné saturé linéaire en C₁-C₅ ou ramifié en C₃-C₅ ou insaturé en C₂-C₅, ou un groupe phényle
R₃ désigne un atome d'hydrogène ; un groupe hydrocarboné saturé linéaire en C₁-C₅ ou ramifié en C₃-C₅ ou insaturé en C₂-C₅, ; un groupe phényle ; un groupe acétyle.
Lorsque Y=NR1, R et R₁ peuvent former un cycle choisi parmi pyrrolidine, pyrroline, pipéridine, pipérazine, morpholine, thiomorpholine, azépine ;
n = 0 ou 1 ou 2 ,
ainsi que leurs sels, leurs chélates, leurs solvates et leurs isomères optiques.

Les sels des composés décrits dans la présente invention comprennent des sels non toxiques conventionnels desdits composés tels que ceux formés à partir d'acides organiques ou minéraux. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.
Les sels de bases organiques ou minérales telles que les sels de triéthanolamine, d'aminopropanediol, de sodium, de zinc.

Les solvates acceptables pour l'usage non thérapeutique des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Les isomères optiques sont notamment les énantiomères et les diastéréoismères.

De façon préférentielle, les groupes alcoxy sont linéaires en C₁-C₄ et plus préférentiellement méthoxy, éthoxy, propoxy, butoxy et encore plus préférentiellement méthoxy.

De façon préférentielle, les groupes hydrocarbonés sont des alkyles linéaires ou ramifiés et peuvent être choisis parmi : méthyle, éthyle, propyle, isopropyle, butyle, isobutyle, tertio-butyle pentyle, hexyle, heptyle octyle, nonyle, decyle, undecyle, docecyle, tridecyle, tetradecyle, pentadecyle, hexadecyle, heptadecyle, octadecyle, nonadecyle, eicosyle.

De façon plus préférentielle, les groupes hydrocarbonés sont des radicaux alkyles linéaires ou ramifiés saturés en C₁-C₈ : méthyle, éthyle propyle isopropyle butyle isobutyle, tertio-butyle, pentyle, hexyle, heptyle, octyle.

De préférence, les composés de formule (I) ont les significations suivantes :
Y désigne S, O, NR₁
R₁ désigne un atome d'hydrogène ; un groupe hydrocarboné alkyle saturé linéaire en C₁-C₁₀ ou ramifié en C₃-C₁₀ ;
R désigne un atome d'hydrogène ; un groupe hydrocarboné alkyle saturé linéaire en C₁-C₂₀ ou ramifié en C₃-C₂₀; un groupe phényle éventuellement substitué par un ou plusieurs hydroxyles et/ou par un ou plusieurs radicaux alcoxy en C₁-C₃ ; un groupe alkyle saturé en C₁-C₅ possédant un substituant phényle éventuellement substitué par un ou plusieurs hydroxyles et/ou par un ou plusieurs radicaux alcoxy en C₁-C₃ ; un groupe hydrocarboné alkyle linéaire en C1-C5 substitué par un ou plusieurs groupes identiques ou non choisis parmi OR₂, SR₂, NR₂R₃, COOR₂ dans lesquels:
R₂ désigne un atome d'hydrogène ou un groupe hydrocarboné saturé linéaire en C₁-C₅ ou ramifié en C₃-C₅ ou insaturé en C₂-C₅,
R₃ désigne un atome d'hydrogène ; un groupe hydrocarboné saturé linéaire en C₁-C₅ ou ramifié en C₃-C₅ ; un groupe phényle ; un groupe acétyle ;
lorsque Y=NR1, R et R₁ peuvent former un cycle pyrrolidine,
n = 0 ou 1 ou 2
ainsi que leurs sels d'acide ou de base, leurs chélates, leurs solvates et leurs isomères optiques.

Préférentiellement, les composés de formule (I) ont les significations suivantes :
Y désigne O ou NR₁ ;
R₁ désigne un atome d'hydrogène ; un groupe hydrocarboné alkyle saturé linéaire en C₁-C₁₀ ou ramifié en C₃-C₁₀ ;
R désigne un atome d'hydrogène ; un groupe hydrocarboné alkyle saturé linéaire en C₁-C₁₀ ou ramifié en C₃-C₁₀ ; un groupe phényle éventuellement substitué par un ou plusieurs hydroxyles et/ou par un ou plusieurs radicaux méthoxy ; un groupe hydrocarboné alkyle saturé en C₁-C₃ possédant un substituant phényle éventuellement substitué par un ou plusieurs hydroxyles et/ou par un ou plusieurs radicaux méthoxy ; un groupe hydrocarboné alkyle linéaire en C1-C4 substitué par un ou plusieurs groupes identiques ou non choisis parmi OR₂, SR₂, NR₂R₃, COOR₂ dans lesquels :
R₂ désigne un atome d'hydrogène ou un groupe hydrocarboné saturé linéaire en C₁-C₅ ou ramifié en C₃-C₅ ,
R₃ désigne un atome d'hydrogène ; un groupe hydrocarboné saturé linéaire en C₁-C₅ ou ramifié en C₃-C₅ ;
n = 0 ou 1 ou 2
ainsi que leurs sels d'acide ou de base, leurs chélates, leurs solvates et leurs isomères optiques.

Plus préférentiellement, les composés de formule (I) ont les significations suivantes :
Y désigne NR₁
R₁ désigne un atome d'hydrogène ; un groupe hydrocarboné alkyle saturé linéaire en C₁-C₄;
R désigne un atome d'hydrogène ; un groupe hydrocarboné alkyle saturé linéaire en C₁-C₁₀ ou ramifié en C₃-C₁₀ ; un groupe phényle ; un groupe alkyle saturé linéaire en C₁-C₄ substitué par un phényle substitué ou non par un ou plusieurs groupes identiques ou non choisis parmi OH, OMe ; un groupe hydrocarboné alkyle linéaire en C1-C4 substitué par un ou plusieurs groupes identiques ou non choisis parmi OH, NHAc, SR2, COOR2 avec R 2 étant un hydrogène ou un groupe alkyle linéaire en C1-C4 ;
n = 0 ou 1 ou 2
ainsi que leurs sels d'acide ou de base, leurs chélates, leurs solvates et leurs isomères optiques.

Préférentiellement, les composés de formule (I) ont les significations suivantes :
Y désigne NH
R désigne un atome d'hydrogène ; un groupe hydrocarboné alkyle saturé linéaire en C₁-C₁₀ ou ramifié en C₃-C₁₀ ; un groupe phényle ; un groupe alkyle saturé linéaire en C₁-C₄ substitué par un phényle substitué ou non par un ou plusieurs groupes identiques ou non choisis parmi OH, OMe ; un groupe hydrocarboné alkyle linéaire en C1-C4 substitué par un ou plusieurs groupes identiques ou non choisis parmi OH, NHAc, SR2, COOR2 avec R 2 étant un hydrogène ou un groupe alkyle linéaire en C1-C4 ;
n = 0 ou 1 ou 2 ;
ainsi que leurs sels d'acide ou de base, leurs chélates, leurs solvates et leurs isomères optiques.

Préférentiellement, les composés de formule (I) ont les significations suivantes :
Y désigne NH
R désigne un atome d'hydrogène ; un groupe hydrocarboné alkyle saturé linéaire en C₁-C₁₀ ou ramifié en C₃-C₁₀ ;
n = 0 ou 1 ou 2 ;
ainsi que leurs sels d'acide ou de base, leurs chélates, leurs solvates et leurs isomères optiques.

De façon préférentielle, Y = O, NR₁.

De façon plus préférentielle, Y = NR₁.

De façon encore plus préférentielle, Y = NH .

De façon très préférentielle, R = H ou un radical alkyle en C₁-C₈.

Parmi les composés de formule (I), on utilisera de préférence les composés suivants

| **N°** | **Structure** | **Nom Chimique** |
|---|---|---|
| 1 | 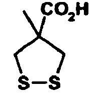 | Acide 4-méthyl-1,2-d ith iolane-4-carboxyl iq ue |
| 2 | 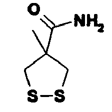 | 4-méthyl-1,2-dithiolane-4-carboxamide |
| 3 | 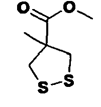 | 4-méthyl-1,2-dithiolane-4-carboxylate de méthyle |
| 4 | 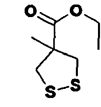 | 4-méthyl-1,2-dithiolane-4-carboxylate d'éthyle |
| 5 | 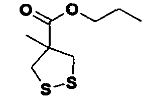 | 4-méthyl-1,2-dithiolane-4-carboxylate de propyle |
| 6 | 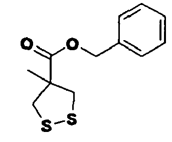 | 4-méthyl-1,2-dithiolane-4-carboxylate de benzyle |
| 7 | 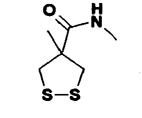 | N-méthyl 4-méthyl-1,2-dithiolane-4-carboxamide |
| 8 | 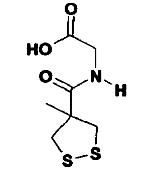 | Acide {[(4-méthyl-1,2-dithiolan-4-yl)carbonyl]aminol acétique |
| 9 | 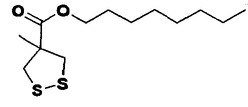 | 4-méthyl-1,2-dithiolane-4-carboxylate d'octyle |
| 10 | 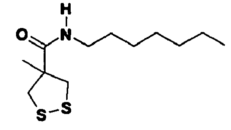 | N-heptyl-4-méthyl-1,2-dithiolane-4-carboxamide |
| 11 | 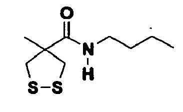 | N-butyl-4-méthyl-1,2-dithiolane-4-carboxamide |
| 12 | 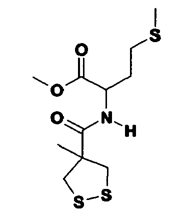 | 2-{[(4-méthyl-1,2-dithiolan-4-yl)carbonyl]amino}-4-(méthylsulfanyl)butanoate de méthyle |
| 13 | 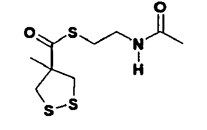 | S-[2-(acétylamino)éthyl] 4-méthyl-1,2-dithiolane-4-carbothioate |
| 14 | 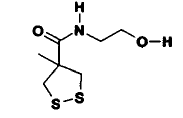 | N-(2-hydroxyéthyl)-4-méthyl-1,2-dithiolane-4-carboxamide |
| 15 | 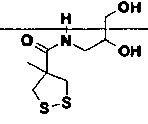 | N-(2,3-dihydroxypropyl)-4-méthyl-1,2-dithiolane-4-carboxamide |
| 16 | 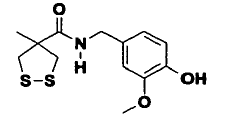 | N-(4-hydroxy-3-méthoxybenzyl)-4-méthyl-1,2-dithiolane-4-carboxamide |
| 17 | 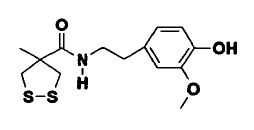 | N-[2-(4-hydroxy-3-méthoxyphenyl)éthyl]-4-méthyl-1,2-dithiolane-4-carboxamide |
| 18 | 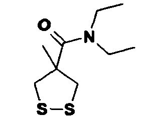 | N,N-diéthyl-4-méthyl-1,2-diithiolane-4-carboxamide |
| 19 | 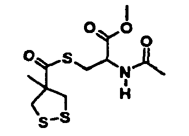 | 2-(acétylamino)-3-{[(4-méthyl-1,2-dithiolan-4-yl)carbonyl]sulfanyl}propano ate de méthyle |
| 20 | 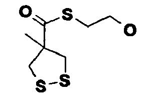 | S-(2-hydroxyéthyl) 4-méthyl-1,2-dithiolane-4-carbothioate |
| 21 | 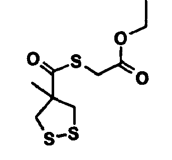 | {[(4-méthyl-1,2-dithiolan-4-yl)carbonyl]sulfanyl}acétate d'éthyle |
| 22 | 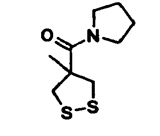 | [(4-méthyl-1,2-dithiolan-4-yl)carbonyl]pyrrolidine |
| 23 | 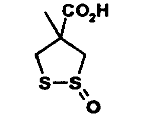 | Acide 4-méthyl-1,2-dithiolane-1-oxyde-4-carboxylique |
| 24 | 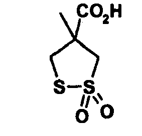 | Acide 4-méthyl-1,2-dithiolane-1,1-dioxyde-4-carboxylique |
| 25 | 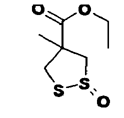 | 4-méthyl-1,2-dithiolane-1-oxyde-4-carboxylate d'éthyle |
| 26 | 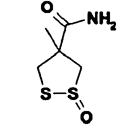 | 4-méthyl-1,2-dithiolane-4-carboxamide 1-oxyde |
| 27 | 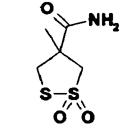 | 4-méthyl-1,2-dithiolane-4-carboxamide 1,1-dioxyde |
| 28 | 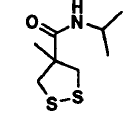 | 4-méthyl-N-(1-methylethyl)-1,2-dithiolane-4-carboxamide |
| 29 | 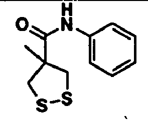 | 4-méthyl-N-phenyl-1,2-dithiolane-4-carboxamide |
| 30 | 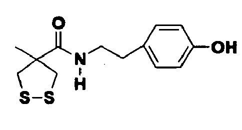 | N-[2-(4-hydroxyphényl)éthyl]-4-méthyl-1,2-dithiolane-4-carboxamide |
| 31 | 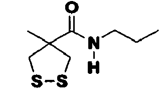 | N-propyl-4-méthyl-1,2-dithiolane-4-carboxamide |
| 32 | 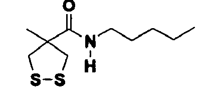 | N-pentyl-4-méthyl-1,2-dithiolane-4-carboxamide |
| 33 | 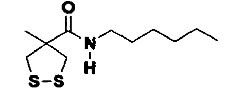 | N-hexyl-4-méthyl-1,2-dithiolane-4-carboxamide |
| 34 | 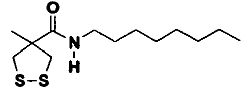 | N-octyl-4-méthyl-1,2-dithiolane-4-carboxamide |
| 35 | 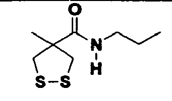 | N-propyl-4-méthyl-1,2-dithiolane-4-carboxamide |
| 36 | 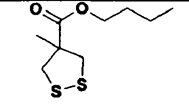 | 4-méthyl-1,2-dithiolane-4-carboxylate de butyle |
| 37 | 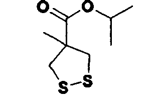 | 4-méthyl-1,2-dithiolane-4-carboxylate d'isopropyle |
| 38 | 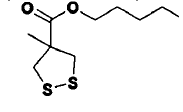 | 4-méthyl-1,2-dithiolane-4-carboxylate de pentyle |
| 39 | 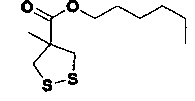 | 4-méthyl-1,2-dithiolane-4-carboxylate d'hexyle |
| 40 | 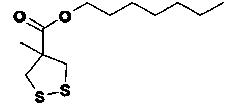 | 4-méthyl-1,2-dithiolane-4-carboxylate d'heptyle |
| 41 | 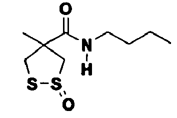 | N-butyl-4-methyl-1,2-dithiolane-4-carboxamide 1-oxide |
| 42 | 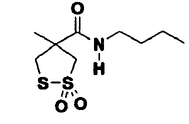 | N-butyl-4-methyl-1,2-dithiolane-4-carboxamide 1-dioxide |

Parmi ces composés, on préfère plus particulièrement les composés suivants :

| **N°** | **Structure** | **Nom Chimique** |
|---|---|---|
| 2 | 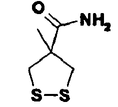 | 4-méthyl-1,2-dithiolane-4-carboxamide |
| 10 | 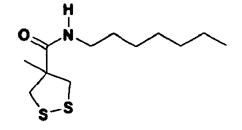 | N-heptyl-4-méthyl-1,2-dithiolane-4-carboxamide |
| 11 | 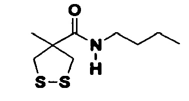 | N-butyl-4-méthyl-1,2-dithiolane-4-carboxamide |
| 23 | 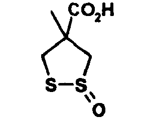 | Acide 4-méthyl-1,2-dithiolane-1-oxyde-4-carboxylique |
| 24 | 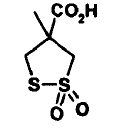 | Acide 4-méthyl-1,2-dithiolane-1,1-dioxyde-4- carboxylique |
| 26 | 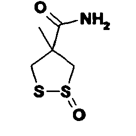 | 4-méthyl-1,2-dithiolane-4-carboxamide 1-oxide |
| 27 | 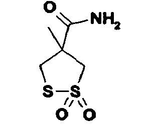 | 4-méthyl-1,2-dithiolane-4-carboxamide 1,1-dioxide |
| 41 | 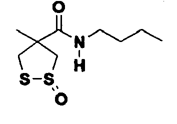 | N-butyl-4-methyl-1,2-dithiolane-4-carboxamide 1-oxide |
| 42 | 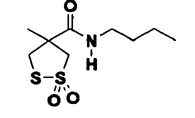 | N-butyl-4-methyl-1,2-dithiolane-4-carboxamide 1-dioxide |

ainsi que leurs sels d'acide ou de base, leurs isomères optiques, leurs solvates.

Certains des composés conformes à l'invention sont connus en soi. Il s'agit des composés 1 à 8 suivants :

| **N°** | **Structure** | **Nom Chimique** | **CAS** |
|---|---|---|---|
| 1 | 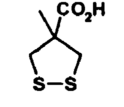 | Acide 4-méthyl-1,2-dithiolane-4-carboxyl iq ue | 208243-72-5 |
| 2 | 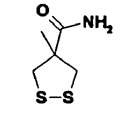 | 4-méthyl-1,2-dithiolane-4-carboxamide | 208243-73-6 |
| 3 | 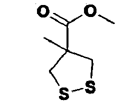 | 4-méthyl-1,2-dithiolane-4-carboxylate de méthyle | 208243-88-3 |
| 4 | 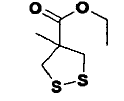 | 4-méthyl-1,2-dithiolane-4-carboxylate d'éthyle | 208243-89-4 |
| 5 | 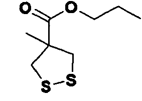 | 4-méthyl-1,2-dithiolane-4-carboxylate de propyle | 208243-90-7 |
| 6 | 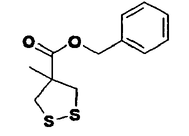 | 4-méthyl-1,2-dithiolane-4-carboxylate de benzyle | 208243-73-6 |
| 7 | 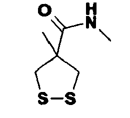 | N-methyl 4-methyl-1,2-dithiolane-4-carboxamide | 208243-91-8 |
| 8 | 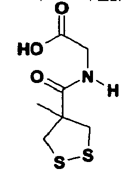 | acide {[(4-methyl-1,2-dithiolan-4-yl)carbonyl]amino} acétique | 208243-74-7 |

Ces derniers ont été décrits dans la demande WO98/23606 et utilisés en pharmacologie comme agents pour réduire le glucose ou les lipides dans le sang

Les composés de formule (I) peuvent être obtenus suivant les voies décrites ci-dessous et documentées
- dans la revue de Lene Teuber, Sulfur reports, 9(4), 257-349,1990 Naturally occuring 1,2-dithiolanes and 1,2,3-tritianes. Chemical and Biological Properties.
- dans la demande de brevet EP 0 869 126 A1.

A partir de l'acide 2,2 bis(hydroxymethyl)propionique (CAS : 4767-03-7), par fonctionnalisation des hydroxyles en groupements partants X (alkyle ou aryles sulfonates tels que mésylates ou tosylates ou halogènes tels que l'iode, le brome ou le chlore) suivie de l'introduction du soufre selon le schéma réactionnel suivant :

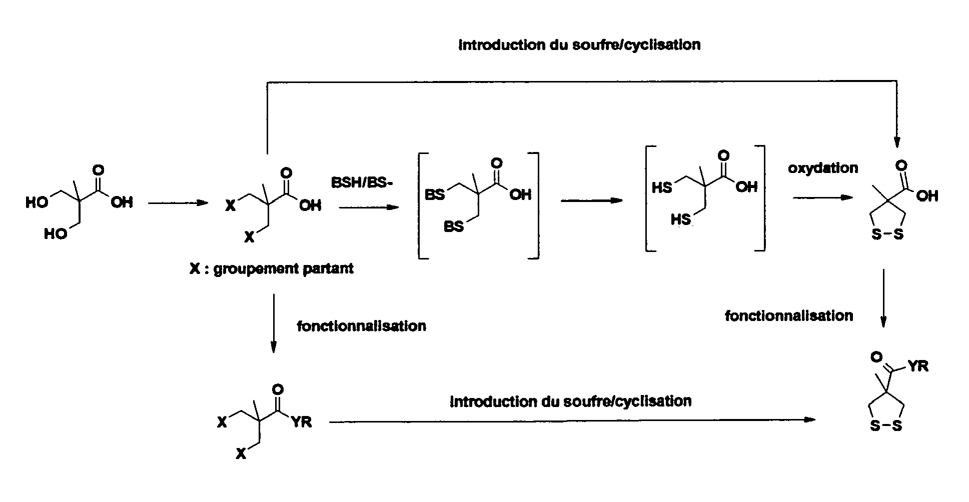

Cette introduction du souffre peut s'effectuer :
(i) en une étape à l'aide de disulfure de métaux (comme le Na₂S₂) ou des sels de tétrathiomolybdates dans des solvants polaires protiques ou aprotiques (par exemple eau, DMF, méthanol, acétonitrile) pour conduire au dithiolane.
(ii) ou en deux étapes en formant un intermédiaire di-thiol, qui en présence d'un oxydant (oxygène, DMSO, FeCl₃, I₂, Br₂, iodure de sodium, trifluoroacetates de thallium, triflates d'argent, eau oxygénée, iodate et periodate de sodium, hypochlorite de sodium, ferricyanide de potassium, oxyde de chrome), en milieu neutre ou basique conduit à la formation du dithiolane. Dans ce cas, le di-thiol est obtenu par transformation (en milieu basique ou acide) dans un solvant polaire ou apolaire d'une espèce intermédiaire via des dérivés de l'acide thioacétique CH₃COSH (en présence de base), par de la thiourée ou du NaSH, par la formation de dithiosulfoantes (sels de Bunte).

La fonctionnalisation de l'acide carboxylique COOH en fonction COYR peut s'effectuer suivant les méthodes classiques d'activation des acides (décrites dans Comprehensive Organic Transformation de R. LAROCK Wiley VCH Ed. dans le chapitre Interconversion of nitriles, carboxylic acids and derivatives). De préférence les méthodes utilisées privilégient le passage au chlorure d'acide (par utilisation de chlorure de thionyle ou d'oxalyle, ou de 1-chloro-n.n.2-trimethyl-1-propenamine) ou par la formation d'anhydride mixte (à l'aide de chloroformiate d'alkyles) ou l'utilisation de carbodiimides ou de diéthylcyanophosphate (Phosphorus in organic synthesis-XI, Amino acids and peptides-XXI, Reaction of diethyl phosphorocyanidate with carboxylic acids. A new synthesis of carboxylic esters and amides, Tetrahedron, 32, 1976, 2211-2217). *

Les solvants utilisées peuvent être polaires ou apolaires, protiques ou aprotiques (par exemple toluène, dichlorométhane, THF, DMF, acétonitrile, eau, méthanol, isopropanol)

Toutes ces réactions peuvent s'effectuer à des températures comprises entre - 20 et 100°C.

L'obtention des produits d'oxydation des atomes de soufre des dithiolanes de formule (I) (n différent de zéro) peut s'effectuer selon le schéma réactionnel suivant : 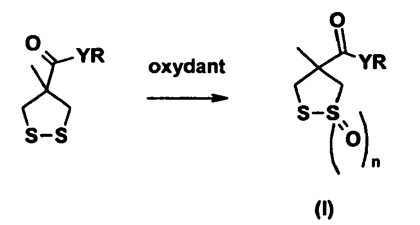 à l'aide d'oxydants tels que l'oxygène, l'eau oxygénée, le DMSO, le périodate de sodium, les peracides organiques, les persulfates inorganiques, les permanganates inorganiques en présence ou non de catalyseur (par exemple Na2WO4, MoO2Cl2, trichloro oxo bis triphenylphosphine rhenium). Les différentes étapes d'oxydation sont fonction de la stoechiométrie des oxydants utilisés. Les solvants utilisables peuvent être l'eau, l'acétone, le dichlorométhane, le méthanol.

Ces oxydations ont été décrites dans les documents suivants :
- Oxydation of 1,2-Dithiolanes, Bernt Lindberg, Göran Bergson, Arkiv För Kemi, 1965, 23(31), 319-333.
- Selective oxydation of sulfides to sulfoxides and sulfones at room temperature using H2O2 and a Mo(VI) salt as catalyst, Kandasamy Jeyakumar, Dillip Kumar Chand, Tetrahedrons Letters 47(2006), 4573-4576
- Rhenium-Catalyzed Oxidation of Thiols and Disulfides with Sulfoxides, Jeffrey B.Arterburn, Marc C.Perry, Sherry L.Nelson, Benjamin R.Dible, Mylena S. Holguin, J. Am. Soc, 119, 9309-9310, 1997

Avantageusement, le composé 1 peut être obtenu suivant la voie décrite ci-dessous à partir de l'acide dichloro-pivalique suivant un procédé en un pot, finalisé par un précipitation.

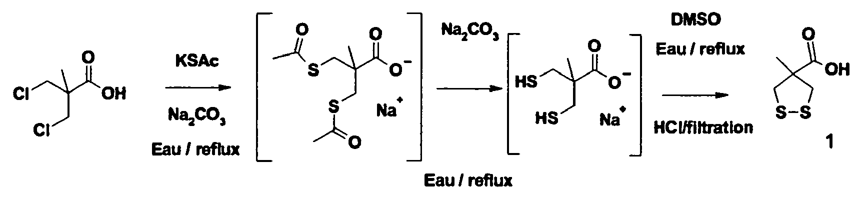

Avantageusement, le composé 2 peut être obtenu à partir du composé 1, préférentiellement à l'aide de chloroformiate d'isobutyle ou de chlorure d'oxallyle.

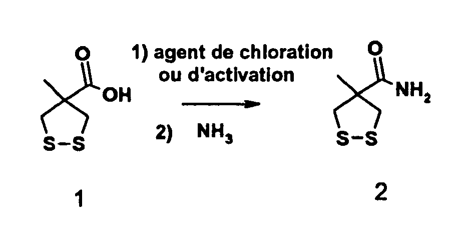

Les composés selon l'invention trouvent une application toute particulière dans le domaine cosmétique ou pharmaceutique, en particulier dermatologique.

Ils peuvent être présents, seuls ou en mélange, dans les compositions cosmétiques ou pharmaceutiques, en une quantité qui peut être comprise entre 0,01 et 10% en poids, de préférence entre 0,1 à 5% en poids, notamment de 0,5 à 3% en poids, par rapport au poids total de la composition.
La composition comprend en outre un milieu physiologiquement acceptable, qui sera préférentiellement un milieu cosmétiquement ou pharmaceutiquement, notamment dermatologiquement, acceptable, c'est-à-dire sans odeur, couleur ou aspect désagréable, et qui ne génère pas de picotement, tiraillement ou rougeur inacceptable pour l'utilisateur. En particulier la composition est adaptée à une application topique sur la peau.
Par milieu physiologiquement acceptable, on comprend un milieu compatible avec les matières kératiniques d'êtres humains comme la peau du corps ou du visage, les lèvres, les muqueuses, les cils, les ongles, le cuir chevelu et/ou les cheveux.

La composition selon l'invention peut alors comprendre tous les adjuvants usuellement employés dans le domaine d'application envisagée.
On peut notamment citer l'eau; les solvants organiques, notamment les alcools en C1-C6 et les esters d'acide carboxylique en C2-C10; les huiles carbonées et/ou siliconées, d'origine minérale, animale et/ou végétale; les cires, les pigments, les charges, les colorants, les tensioactifs, les émulsionnants, les co-émulsionnants; les actifs cosmétiques ou dermatologiques, les filtres UV, les polymères, les gélifiants hydrophiles ou lipophiles, les épaississants, les conservateurs, les parfums, les bactéricides, les céramides, les absorbeurs d'odeur, les antioxydants.
Ces éventuels adjuvants peuvent être présents dans la composition à raison de 0,001 à 80% en poids, notamment 0,1 à 40% en poids, par rapport au poids total de la composition. Ces adjuvants, selon leur nature, peuvent être introduits dans la phase grasse ou dans la phase aqueuse de la composition, ou dans des vésicules lipidiques. En tout état de cause, ces adjuvants, ainsi que leurs proportions, seront choisis par l'homme du métier de manière telle que les propriétés avantageuses des composés selon l'invention ne soient pas, ou substantiellement pas, altérées par l'adjonction envisagée.

Comme actifs, il sera avantageux d'introduire dans la composition utilisée selon l'invention au moins un composé choisi parmi: les agents desquamants; les agents apaisants, les agents photoprotecteurs organique ou inorganique, les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaire; les agents agissant sur la microcirculation; les agents agissant sur le métabolisme énergétique des cellules; et leurs mélanges.

Des exemples de tels composés additionnels sont: le rétinol et ses dérivés tels que le palmitate de rétinyle; l'acide ascorbique et ses dérivés tels que l'ascorbyl phosphate de magnésium et le glucoside d'ascorbyle; le tocophérol et ses dérivés tels que l'acétate de tocophéryle; l'acide nicotinique et ses précurseurs tels que la nicotinamide; l'ubiquinone; le glutathion et ses précurseurs tels que l'acide L-2-oxothiazolidine-4-carboxylique; les extraits de plantes et notamment les protéines végétales et leurs hydrolysats, ainsi que les phytohormones; les extraits marins tels que les extraits d'algues; les extraits bactériens; les sapogénines telles que la diosgénine et les extraits de Wild Yam en contenant; les céramides; les hydroxyacides tels que l'acide salicylique et l'acide n-octanoyl-5-salicylique; le resvératrol; les oligopeptides et pseudodipeptides et leurs dérivés acylés; les sels de manganèse et de magnésium, en particulier les gluconates; et leurs mélanges.

Par agent desquamant, on entend tout composé capable d'agir :
- soit directement sur la desquamation en favorisant l'exfoliation, tel que les β -hydroxyacides, en particulier l'acide salicylique et ses dérivés (dont l'acide n-octanoyl 5-salicylique); les α-hydroxyacides, tels que les acides glycolique, citrique, lactique, tartrique, malique ou mandélique; l'urée; l'acide gentisique; les oligofucoses; l'acide cinnamique; l'extrait de Saphora japonica; le resvératrol;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux: l'EDTA; l'acide N-acyl-N,N',N' éthylène diaminetriacétique; les composés aminosulfoniques et en particulier l'acide (N-2 hydroxyéthylpiperazine-N-2-éthane) sulfonique (HEPES); les dérivés de l'acide 2-oxothiazolidine-4-carboxylique (procystéine); les dérivés d'acides alpha aminés de type glycine (tels que décrits dans EP-0 852 949, ainsi que le méthyl glycine diacétate de sodium commercialisé par BASF sous la dénomination commerciale TRILON M); le miel; les dérivés de sucre tels que l'O-octanoyl-6-D-maltose et la N-acétyl glucosamine.
Les agents desquamants sont généralement présents dans la composition selon l'invention dans des proportions allant de 0,01 à 15% en poids, de préférence allant de 0,1 à 10% en poids, par rapport au poids total de la composition.

Comme agents apaisants utilisables dans la composition selon l'invention, on peut citer: les triterpènes pentacycliques et les extraits de plantes (par exemple Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3-stéaroyloxy glycyrrhetique), l'acide ursolique et ses sels, l'acide oléanolique et ses sels, l'acide bétulinique et ses sels, un extrait de Paeonia suffruticosa et/ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier musca, de cassis, d'ecchium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa moniera, les phytostérols, la cortisone, l'hydrocortisone, l'indométhacine et la beta méthasone.
Les agents apaisants sont généralement présents dans la composition selon l'invention dans des proportions allant de 0,01 à 15% en poids, de préférence allant de 0,1 à 10% en poids, par rapport au poids total de la composition.

Les agents photoprotecteurs organiques sont notamment choisis parmi les anthranilates; les dérivés cinnamiques; les dérivés de dibenzoylméthane; les dérivés salicyliques, les dérivés du camphre; les dérivés de triazine tels que ceux décrits dans les demandes de brevet US4367390, EP863145, EP517104, EP570838, EP796851, EP775698, EP878469, EP933376, EP507691, EP507692, EP790243, EP944624; les dérivés de la benzophénone; les dérivés de β,β-diphénylacrylate, les dérivés de benzotriazole; les dérivés de benzalmalonate; les dérivés de benzimidazole; les imidazolines; les dérivés bis-benzoazolyle tels que décrits dans les brevets EP669323 et US2,463,264; les dérivés de l'acide p-aminobenzoïque (PABA); les dérivés de méthylène bis-(hydroxyphényl benzotriazole) tels que décrits dans les demandes US5237071, US5166355, GB2303549, DE19726184 et EP893119; et les polymères filtres et silicones filtres tels que ceux décrits notamment dans la demande WO-93/04665; les dimères dérivés d'α-alkylstyrène tels que ceux décrits dans la demande de brevet DE19855649.
Les agents photoprotecteurs inorganiques peuvent notamment être choisis parmi des pigments ou des nanopigments (taille moyenne des particules primaires généralement comprise entre 5 nm et 100 nm, de préférence entre 10 nm et 50 nm) d'oxydes métalliques, enrobés ou non, comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.
Les agents photoprotecteurs sont généralement présents dans la composition selon l'invention dans des proportions allant de 0,1 à 20% en poids, de préférence allant de 0,2 à 15% en poids, par rapport au poids total de la composition.

Cette composition peut se présenter sous toutes les formes galéniques normalement utilisées dans le domaine cosmétique ou pharmaceutique, et notamment sous forme d'une solution aqueuse ou hydroalcoolique, éventuellement gélifiée, d'une dispersion du type lotion éventuellement biphasée, d'une émulsion huile-dans-eau ou eau-dans-huile ou multiple ((E/H/E ou H/E/H par exemple), d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non ionique; de gel aqueux ou huileux. Ces compositions sont préparées selon les méthodes usuelles. On préfère utiliser selon cette invention une composition sous la forme d'une émulsion notamment huile-dans-eau.
La composition peut être plus ou moins fluide et avoir l'aspect d'une crème blanche ou colorée, d'une pommade, d'un lait, d'une lotion, d'un sérum, d'une pâte, d'un gel, d'une mousse. Elle peut éventuellement être appliquée sous forme d'aérosol. Elle peut également se présenter sous forme solide, en particulier sous forme de stick.
Lorsque la composition est une émulsion, la proportion de la phase grasse peut aller de 5 à 80% en poids, de préférence de 8 à 50% en poids par rapport au poids total de la composition. L'émulsionnant et le co-émulsionnant peuvent être présents en une proportion allant de 0,3 à 30% en poids, et de préférence de 0,5 à 20% en poids, par rapport au poids total de la composition.

La composition utilisée selon l'invention peut constituer une composition de soin de la peau, et notamment une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un fond de teint fluide, un lait de démaquillage, un lait corporel de protection ou de soin, un lait anti-solaire; une lotion, gel ou mousse pour le soin de la peau, comme une lotion de nettoyage.

L'invention est illustrée plus en détail par les exemples non limitatifs suivants.

### Exemples de synthèse:

### Exemple 1 : synthèse de l'acide 4-methyl-1,2-dithiolane-4-carboxylique (composé 1)

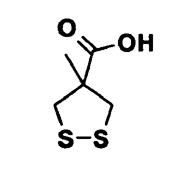

8g d'acide dichloropivalique sont placés dans un tricol de 250ml surmonté d'un réfrigérant et d'une ampoule de coulée. L'acide est dissout dans 80ml d'eau, en ajoutant lentement 4,6g de Na₂CO₃. Une solution de 10,7 g de thioacétate de potassium est additionnée goutte à goutte, le milieu réactionnel est porté au reflux. 14,9 g de Na₂CO₃ sont ajoutés et le milieu chauffé de nouveau au reflux. Après disparition du produit de départ, 7,3 ml de DMSO sont ajoutés avant de chauffer au reflux. Le dithiolane est obtenu après acidification par précipitation et séchage sous vide du solide. On a obtenu un solide jaune pâle
RMN 1H (400 MHz, DMSO-d6): δ ppm 3,69 (d, 2H), 2,95 (d, 2H), 1,53 (s, 3H), ESI- :
[(M, H) -] = 163 m/z

### Exemple 2: Synthèse de l'octyl 4-methyl-1,2-dithiolane-4-carboxylate (composé 9)

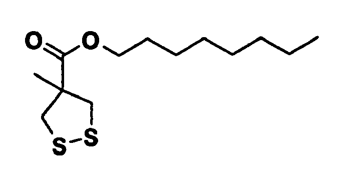

Dans un tricol de 100 ml, sont chargés sous argon 1g d'acide (24) puis 0,8 ml du 1-chloro-N,N-2-triméthyl-propenylamine (27) à l'aide d'une seringue dans 20 ml de dichlorométhane le mélange est agité pendant 1 h puis introduit goutte à goutte par une ampoule de coulée dans un milieu réactionnel à -5°C contenant 1.28ml de triéthylamine, 0,96 ml d'octanol et 20 ml de dichlorométhane. On laisse agiter. Le milieu réactionnel est ensuite lavé à l'eau (3 X30 ml). La phase aqueuse est extraite avec 3X10ml d'AcOEt . Les phases organiques réunies sont lavées avec 30ml de solution aqueuse saturée de NaCl puis séchées sur Na₂SO₄, filtrées puis concentrées sous vide (500mbar, T=40°C) au rotavapor. Le brut obtenu est une huile jaune (m=1,25g). La purification se fait par chromatographie flash sur une colonne de silice (mSiO₂=40g-Eluant par gradient Heptane/AcOEt : 100/0 puis 98/2.)
Après concentration des fractions au rotavapor (P=100 mbar, T=40°C), on a obtenu 1,08 g d'huile jaune.
Rendement =66% ; Rf (ester)= 0,16 (Eluant : Cyclohexane) ;
RMN 1H (400 MHz, DMSO-d6): δ ppm 4,08 (t, 2H), 3,57 (d, 2H), 3,02 (d, 2H), 1,58 (m, 2H), 1,40 (s, 3H), 1.29 (m, 10H), 0,86 (t, 3H)
MS m/z (M+, 277 ; M+23, 299).

Les manipulations suivantes ont été faites dans les mêmes conditions précédemment décrites, seul le nucléophile varie.

### Exemple 3 : Synthèse du S-[2-(acetylamino)ethyl] 4-methyl-1,2-dithiolane-4-carbothioate (Composé 13)

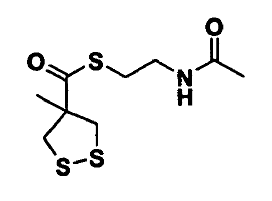

Méthode identique à l'exemple 2: le nucléophile utilisé est la N-acétylcystéamine (0,64ml).
La purification se fait par chromatographie flash sur une colonne de silice (mSiO2=40g- Eluant par gradient linéaire DCM/MeOH : 100/0 puis 98/2.)
Après concentration des fractions au rotavapor (P=200 mbar, T=40°C), on a obtenu 0,32 g d'un mélange comprenant le composé attendu et de N,N-2-triméthyl-propionamide . Après évaporation sous vide, on a obtenu le composé final attendu se présentant sous la forme d'un liquide jaune épais.
Rendement=10% ; Rf (attendu)= 0,3 ; Eluant : DCM/MeOH : 95/5 ; RMN 1H (DMSO-d6): δ ppm 8,03 (t, NH), 3,57 (d, 2H), 3,18 (d.t 2H), 3,10 (d, 2H), 2,.96 (m, 2H), 1,79 (s, 3H), 1,43 (s, 3H) ; MS m/z (M+, 266 ; M+23, 288).

### Exemple 4: Synthèse du N-(2-hydroxyethyl)-4-methyl-1,2-dithiolane-4-carboxamide (Composé 14)

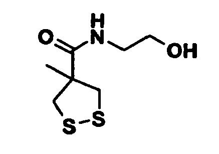

Méthode identique à l'exemple 2 : le nucléophile utilisé est l'éthanolamine (0,36ml). Après filtration du milieu réactionnel, on obtient un brut, huile jaune (m=1,850g)
La purification se fait par chromatographie flash sur une colonne de silice (Eluant par gradient linéaire DCM/MeOH : 100/0 puis 98/2.
Après concentration des fractions au rotavapor (P=500 mbar, T=40°C), on a obtenu 800 mg d'une huile jaune (composé pur) R=65%
Rf (attendu)= 0,43 ; Eluant : DCM/MeOH : 9/1 ; RMN 1H (DMSO-d6): δ ppm 7,80 (t, NH), 4,64 (t, OH), 3,53 (d. 2H), 3,40 (d.t, 2H), 3,14 (m, 2H), 2,99 (d, 2H), 1,34 (s, 3H) ; MS m/z (M+, 208 ; M+23, 230).

### Exemple 5 : Synthèse du 4-methyl-1,2-dithiolane-4-carboxamide (Composé 2)

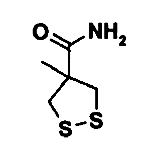

Méthode (ex5-a) identique à l'exemple 2 : le nucléophile utilisé est l'ammoniaque dans l'isopropanol (9,5ml). Après filtration du milieu réactionnel, on obtient un brut, huile jaune (m=1.853g).

La purification se fait par chromatographie flash sur une colonne de silice (Eluant : DCM).

Après concentration des fractions au rotavapor (P=600 mbar, T=40°C), on obtient 500 mg d'attendu pur, solide jaune Rendement =52%.

Alternativement, méthode (ex5-b), à une solution de 1g de composé 1 dans le THF avec 1,2 équivalent de triéthylamine, sont ajoutés à 0°C 1,2 équivalent de chloroformiate d'isobutyle. Après 2 heures à température ambiante, le milieu réactionnel est ajouté à de l'ammoniac en solution refroidie, soit dans l'eau à 28% soit 2N dans l'isopropanol. Le milieu est agité à température ambiante le temps nécessaire avant concentration sous vide. Le brut est alors repris dans le toluène pour conduire par précipitation au composé 2. Rendement = 60%

Rf (attendu)= 0,45 ; Eluant : DCM/MeOH : 95/5 ; RMN 1H (DMSO-d6): δ ppm 7,38 (s, NH), 7,13 (s, NH), 3,53 (d. 2H), 2,97 (d, 2H), 1,34 (s, 3H) ; ESI- : [(M, H) -] = 162 m/z ; ESI+ : [(M, Na) +] = 186 m/z ; ESI+ : [(M, H) +] = 164 m/z ; ESI+ : [(M, Na, MeOH) +] = 218 m/z

### Exemple 6 : Synthèse du N-(2,3-dihydroxypropyl)-4-methyl-1,2-dithiolane-4-carboxamide (Composé 15)

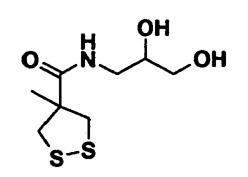

Méthode identique à l'exemple 2 : la quantité d'acide de départ engagé est 0,.25 g et le nucléophile utilisé est le diméthyldioxalaneméthanamine (0.2ml).
On obtient 110mg d'attendu pur, huile jaune Rendement=26%
Rf (attendu)= 0,51 ; Eluant : DCM/MeOH : 95/5 ; RMN 1H (DMSO-d6): δ ppm 7.77 (t, 1H :NH), 3,55 (d.d 4H, H3 :diastéréoisomères), 3,5 (m, 4H : H7 diastéréoisomères), 3,20 (m, 2H : H8 : diastéréoisomères), 3,05 (d.d, 2H:H9 et H9'), 2,99 (d.d, 4H, H5), 1,35 (s, 12H, H10+H11: diastéréoisomères), 0,9 (d, 3H, H6); MS m/z (M+23, 300).
70mg du produit pur protégé sous forme d'acétonide et environ 5 g de résine Dowex sont engagés dans une solution de 3ml d'eau et 2ml de THF. Le mélange réactionnel est agité à TA pendant 20h, puis à 40°C pendant 40h.
Le milieu réactionnel avec de la résine est filtré sous vide et lavé avec 3*10 ml d'eau puis 2X10ml d'EtOH. Le filtrat est ensuite concentré au rotavapor (P=200 mbar, T=40°C), on a obtenu 30 mg d'huile jaune présentant 2 diastéréoisomères.
Rf (attendu)= 0,24 ; Eluant : DCM/MeOH : 9/1 ; RMN 1H (DMSO-d6): δ ppm 7,80 (t, 1H : NH), 4.73 (d, OH), 4.50 (t, OH), 3,55 (d. 4H), 3,4 (m. 2H), 3.,2 (m, 1H), 3.1 (m, 2H), 2,99 (d, 4H), 1,35 (s, 3H) ; MS m/z (M+, 208 ; M+23, 230).

### Exemple 7 : Synthèse du N-heptyl-4-methyl-1,2-dithiolane-4-carboxamide (Composé 10)

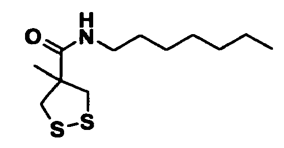

Méthode identique à l'exemple 2 : le nucléophile est 0.22ml de n-Heptylamine.
Le brut obtenu est une huile jaunâtre (m=0,27g).
La purification se fait par chromatographie flash sur une colonne de silice (mSiO₂=12g- Eluant DCM/MeOH : 99/1)
Après concentration des fractions au rotavapor (P=500 mbar, T=40°C), on a obtenu 0.21 g d'une huile jaune (composé 10 pur) Rendement=54%. Rf (attendu)= 0.5 ; Eluant : DCM/MeOH : 99/1 ; RMN 1H (DMSO-d6): δ ppm 7.78 (t, NH), 3,53 (d. 2H), 3.1 (d.t, 2H), 2,97 (d, 2H), 1,41 (t.t. 2H), 1,34 (s, 3H), 1,23 (m, 8H), 0,85 (t, 3H) ; MS m/z (M+, 262 ; M+23, 284)

### Exemple 8: Synthèse du Methyl 2-{[(4-methyl-1,2-dithiolan-4-yl)carbonyl]amino}-4-(methylsulfanyl)butanoate (Composé 12)

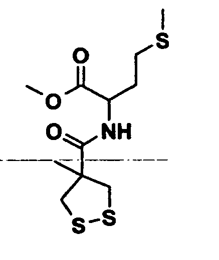

Méthode identique à l'exemple 2 : le nucléophile est la L-méthionine methylester. On a obtenu 140 mg d'une huile jaune (Rendement 7%).
RMN 1H (DMSO-d6): δ ppm 8,13 (d, NH), 4,4 (m. 1H), 3.63 (s, 3H), 3,58 (m, 2H), 3,02 (m, 2H), 2,5 (m, 2H), 2.04 (s, 3H), 1,96 (m, 2H), 1,38 (s, 3H); MS m/z (M+, 310 ; M+23, 332)

### Exemple 9: Synthèse du N-butyl-4-methyl-1,2-dithiolane-4-carboxamide (Composé 11)

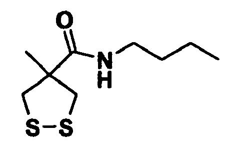

A 1g d'acide 1,2 dithiolane 4-méthyl 4-carboxylique dans 20 ml de THF anhydre sont ajoutés 1,1 équivalent de triéthylamine et 1, équivalent de diéthylcyanophosphate à 0°C. 1,1 équivalent de n-butylamine sont ajoutés à 0°C et le milieu est agité pendant 1 heure avec un retour à température ambiante. Après évaporation et traitement aqueux par extraction, le brut réactionnel reconcentré est purifié sur colonne de silice (éluant dichlorométhane). Après évaporation des fractions d'intérêt on a obtenu une huile jaune.
RMN 1H (DMSO-d6): δ ppm 7,79 (t, NH), 3,53 (d. 2H), 3,1 (d.t, 2H), 2,97 (d, 2H), 1,41 (t.t. 2H), 1,34 (s, 3H), 1,23 (m, 8H), 0,85 (t, 3H) ; MS m/z (M+, 262 ; M+23, 284)
RMN 1H (DMSO-d6): δ ppm 7,79 (t, NH), 3,54 (d. 2H), 3,08 (d.t, 2H), 2,98 (d, 2H), 1,40 (q 2H), 1,34 (s, 3H), 1,27 (m, 4H), 0,87 (t, 3H) ; ESI+ : [(M, Na) +] = 242 m/z

### Exemple 10: Synthèse du 4-methyl-1,2-dithiolane-4-carboxylic acid 1-oxide (Composé 23)

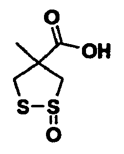

A 100mg d'acide 4-methyl-1,2-dithiolane-4-carboxylique dans 2 ml d'acétone, sont ajoutés 1 équivalent d'eau oxygénée à 30%. Le milieu réactionnel est agité à 20°C pendant une nuit. Après concentration sous vide, on a obtenu quantitativement le thiosulfinate sous forme d'un solide blanc mélange de 2 diastéréoisomères en proportion 70/30.
RMN 1H (DMSO-d6): δ ppm
Diastéréoisomère majoritaire : 4,38 (d, 1H), 3,78 (q, 2H), 3,11 (d, 1H), 1,57 (s, 3H)
Diastéréoisomère minoritaire : 4,36 (d, 1H), 3,96 (d, 1H), 3,.42 (d, 1H), 3,31 (d, 1H), 1,51 (s, 3H)
RMN 13 C(DMSO-d6): δ ppm : 174,95; 174,63; 71,96; 70,85; 58,98; 56,73; 46,53; 45,03; 23,77; 21,96
ESI- : [(M, H) -] = 179 m/z

### Exemple 11 : Synthèse du 4-methyl-1,2-dithiolane-4-carboxylic acid 1,1-dioxide (Composé 24)

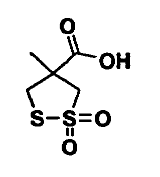

A 100mg d' Acide 4-methyl-1,2-dithiolane-4-carboxylique dans 2 ml d'acétone, sont ajoutés 2 équivalents d'eau oxygénée à 30% et 0,15 équivalent de tungstate de sodium Na2WO4. Le milieu réactionnel est agité à 20°C pendant une nuit. Après filtration et concentration sous vide, le brut a été purifié sur colonne de silice pour conduire au thiosulfonate sous forme d'un solide blanc.
RMN 1H (DMSO-d6): δ ppm 4,14 (d, 1H), 4,05 (d, 1H), 3,69 (d, 1H), 3,66 (d, 1H), 1,51 (s, 3H);
RMN 13 C (DMSO-d6): δ ppm 173,90; 65,86; 50,26; 44,58; 23,59
ESI- : [(M, H) -] = 195 m/z

### Exemple 12: Synthèse du Ethyl 4-methyl-1,2-dithiolane-4-carboxylate (Composé 4)

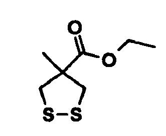

A 1g d' Acide 4-methyl-1,2-dithiolane-4-carboxylique dans 20 ml d'éthanol est ajouté de la résine sulfonique DOWEX 50 WX8 (vendue par Aldrich). Le mélange est porté au reflux pendant 24heures puis filtré et évaporé pour conduire à l'ester éthylique pur.
RMN 1H (DMSO-d6): δ ppm 4,13 (q, 2H), 3,58 (d, 2H), 3,02 (d, 2H), 1,40 (s, 3H), 1,20 (t, 3H)
ESI+ : [(2M, Na) +] = 407 m/z

### Exemple 13 : Synthèse du Ethyl 4-methyl-1,2-dithiolane-4-carboxylate 1-oxide (Composé 25)

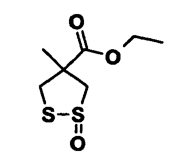

L'oxydation de l'ester d'éthyle s'effectue de la même manière que pour l'acide.
On a obtenu 175 mg d'une huile jaune pâle.
RMN 1H (DMSO-d6): δ ppm
Diastéréoisomère majoritaire : 4,4 (d, 1H), 4,11 (q, 2H), 3,8 (d, 1H), 3,75 (d, 1H), 3,17 (d, 1H), 1.59 (s, 3H), 1.53 (t, 3H)
Diastéréoisomère minoritaire : 4,2 (d, 1H), 4,11 (q, 2H), 3,98 (d, 1H), 3,8 (d, 1H), 3,42 (d, 1H), 3.32 (d, 1H), 1.51 (s, 3H)
RMN 13 C(DMSO-d6): δ ppm 174,95, 174,63, 71,96, 70,85, 58,98, 56,73, 46,53, 45,03, 23,77, 21,96
ESI+ : [(M, Na) +] = 231 m/z ; ESI+ : [(M, Na, MeOH) +] = 263 m/z ; ESI+ : [(2M, Na) +] = 439 m/z
ESI+ : [(M, Na) +] = 231 m/z ; ESI+ : [(M, Na, MeOH) +] = 263 m/z ; ESI+ : [(2M, Na) +] = 439 m/z

### Exemple 14 : Synthèse du 4-methyl-1,2-dithiolane-4-carboxamide 1-oxide (Composé 26)

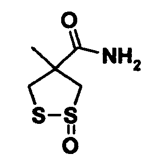

A 100 mg de 4-methyl-1 ,2-dithiolane-4-carboxamide dans 2 ml d'acétone, sont ajoutés 1 équivalent d'eau oxygénée à 30%. Le milieu réactionnel est agité à 20°C pendant une nuit. Après concentration sous vide et purification sur colonne de silice, on obtient le thiosulfinate sous forme d'un solide blanc mélange de 2 diastéréoisomères.
RMN 1H (DMSO-d6): δ ppm
Diastéréoisomère majoritaire : 7,40 (dl, 2H), 4,31 (d, 1H), 3,78 (sl, 2H), 3,04 (d, 1H), 1,49 (s, 3H)
Diastéréoisomère minoritaire : 7,32 (dl, 2H), 4,21 (d, 1H), 3,92 (d, 1H), 3,42 (d, 1H), 3,34 (d, 1H), 1,40 (s, 3H)
ESI- : [(M, H)-]= 178 m/z

### Exemple 15: Synthèse du 4-methyl-1,2-dithiolane-4-carboxamide 1,1-dioxide (Composé 27)

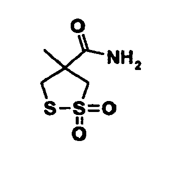

A 100mg 4-methyl-1,2-dithiolane-4-carboxamide dans 2 ml d'acétone, sont ajoutés 2 équivalents d'eau oxygénée à 30% et 0,15 équivalent de tungstate de sodium Na₂WO₄. Le milieu réactionnel est agité à 20°C pendant une nuit. Après filtration et concentration sous vide, le brut est purifié sur colonne de silice pour conduire au thiosulfonate sous forme d'un solide blanc.
RMN 1H (DMSO-d6): δ ppm 7,50 (dl, 2H), 4,21 (d, 1H), 4,08 (d, 1H), 3,66 (d, 1H), 3,59 (d, 1H), 1,49 (s, 3H);
ESI- : [(M, H) -] = 194 m/z

### Exemple 16: Synthèse du N-(4-hydroxy-3-methoxybenzyl)-4-methyl-1,2-dithiolane-4-carboxamide (Composé 16)

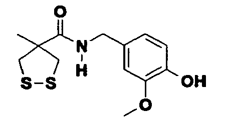

A 24,3 mmol de dithiolane acide, en solution dans 60 ml de dichorométhane, refroidi à 0°C (avec bain de glace) sont ajoutés 24,3 mmol de N-hydroxysuccinimide. Le milieu réactionnel est agité 30 min à 0°C. Une solution de 24,3 mmol de DCC dans 50ml de dichlorométhane est ajouté puis le mélange est agité à 20°C pendant 4 heures. Le milieu réactionnel est filtré et lavé puis le filtrat est évaporé à sec au rotavopor à 40°C sous vide pour conduire au 1-{[(4-methyl-1,2-dithiolan-4-yl)carbonyl]oxylpyrrolidine-2,5-dione. (m=7g, Rdt quantitatif). A 1,58 mmol de 1-{[(4-methyl-1,2-dithiolan-4-yl)carbonyl]oxy}pyrrolidine-2,5-dione, sont ajoutés 10ml de MethylTHF, 3.16mmol de chlorhydrate de 4-(aminomethyl)-2-methoxyphenol et 3,16 mmol de triéthylamine. Après agitation une nuit, le mélange est filtré, rincé avec du MethyITHF puis évaporé. Une chromatographie flash éluant dichloromethane/Méthanol 98/2 permet d'obtenir le composé 16 sous forme d'une huile jaune (Rdt 84%)

RMN 1H (DMSO-d6): δ ppm 1,39 (s,3H) ; 3,03 (d,2H) ; 3,56 (d, 2H), 3,63 (s, 3H, OCH3), 4,21 (d, 2H), 6,64 (dd, 1H,Ar), 6,69 (d,1H, Ar), 6.80 (d, 1H, Ar), 8,31 (t, 1H, NH), 8,79 (s,1H, OH)
ESI+ : [(M, H)+] = 300 m/z

### Exemple 17: Synthèse du N-[2-(4-hydroxy-3-methoxyphenyl)ethyl]-4-methyl-1,2-dithiolane-4-carboxamide (Composé 17)

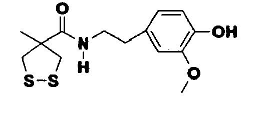

Même méthode que pour le composé 16 avec le chlorhydrate de 4-(2-aminoethyl)-2-methoxyphenol. On a obtenu 320 mg d'un solide jaune (Rdt=64%).
RMN 1H (DMSO-d6): δ ppm 1,31 (s,3H); 2,63 (t, 2H), 2,97(d,2H); 3,24 (m,2H, NCH₂) 3,53 (d, 2H), 3,75 (s, 3H, OCH3), 6,57 (dd, 1H,Ar), 6,67 (d,1H, Ar), 674 (d, 1H, Ar), 8.86(t, 1H, NH), 8,67(s,1H, OH)
ESI+ : [(M, H)+] = 314 m/z

### Exemple 18: Synthèse du N,N-diethyl-4-methyl-1,2-dithiolane-4-carboxamide (Composé 18)

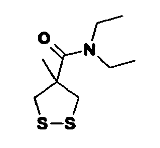

A 2.26mmol de composé 1 sont ajoutés 5 ml de dichloromethane anhydre, 0,1ml de DMF anhydre. Le mélange est refroidi à 0°C avant l'ajout de 2,7 mmol de chlorure d'oxalyl. Le mélange est agité à 20°C avant d'être ajouté à 0°C à un mélange de 2.26mmol de diéthylamine, 5ml de dichloromethane anhydre, 6.8mmol de Diisopropylethylamine. Le milieu réactionnel est agité 3h à 20°C. Lorsque la réaction est terminée, le milieu est dilué dans 50ml de dichlorométhane, puis lavé avec 2x30ml d'eau, 1x50ml de solution saturée de NH4Cl, puis séché sur Na₂SO₄ et évaporé à sec au rotavapor. Après flash chromatographie (éluant heptane/AcOEt) est isolé le composé 18 sous forme d'une huile jaune (Rdt 48%)

RMN 1H (DMSO-d6): δ ppm 1,36 (s,3H) ; 3,15 (d,2H) ; 3,50 (d, 2H), 3 3 (m, 2x2H), 1,.07( m, 2x3H)
ESI+ : [(M, H)+] = 220 m/z

### Exemple 19: Synthèse du Methyl 2-(acetylamino)-3-{[(4-methyl-1,2-dithiolan-4-yl)carbonyl]sulfanyl}propanoate (Composé 19)

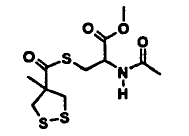

Même méthode que dans l'exemple 18 avec la N-acétylcystéine méthyle ester. On a obtenu 90 mg d'une huile jaune (Rdt 12%).

RMN 1H (DMSO-d6): δ ppm 1,42 (s,3H) ; 1.84(s, 3H, OCH₃), 3,15 (d,2H) ; 3,.56 (d, 2H), 3,38-3,12 (dd, 2H), 3,65 ( s,3H,COCH3), 8,42 (d, 1H, NH)
ESI+ : [(M, H)+] = 324 m/z

### Exemple 20: Synthèse du S-(2-hydroxyethyl) 4-methyl-1,2-dithiolane-4-carbothioate (Composé 20)

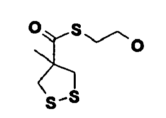

Même méthode que dans l'exemple 18 avec le sulfanyléthanol .
On a obtenu 130 mg d'une huile jaune (Rdt 26%)

RMN 1H (DMSO-d6): δ ppm 1,43 (s,3H) ; 3,1 (d,2H) ; 3,56 (d, 2H), 2,99 (t, 2H:CH2S), 3.48 (q, 2H:CH₂OH)
ESI+ : [(M, Na)+] = 247 m/z

### Exemple 21 : Synthèse du Ethyl {[(4-methyl-1,2-dithiolan-4-yl)carbonyl]sulfanyl}acetate (Composé 21)

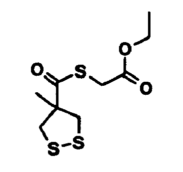

Même méthode que dans l'exemple 17 avec le thioglycolate d'éthyle .
On a obtenu 30 mg d'une huile quasi incolore (Rdt 7%)

RMN 1H (DMSO-d6): δ ppm 1,53 (s,3H) ;1,28 (t, 3H), 2.,99 (d,2H) ; 3,65 (d, 2H), 3,71 (s, 2H, SCH2), 4,20 (q, 2H)

### Exemple 22 : Synthèse du [(4-methyl-1,2-dithiolan-4-yl)carbonyl]pyrrolidine (Composé 22)

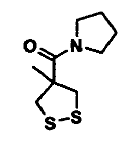

Même méthode que pour l'exemple 5-b avec la pyrrolidine
On a obtenu 130 mg d'un solide jaune (Rdt 38%)
RMN 1H (DMSO-d6): δ ppm 1,36 (s,3H) ; 1,81 (m, 2x2H); 3,10 (d,2H) ; 3,30 (m, 2x2H); 3,58 (d, 2H),
ESI+ : [(M, H)+] = 218 m/z

### Exemple 23: Synthèse du 4-methyl-N-(1-methylethyl)-1,2-dithiolane-4-carboxamide (Composé 28)

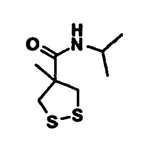

Même méthode que dans l'exemple 18 avec l'isopropylamine.
On a obtenu 250 mg d'un solide beige (Rdt 54%)

RMN 1H (DMSO-d6): δ ppm 1,06 (d, 2x3H); 1,33 (s,3H, Hc) ; 2,99 (d,2H, Hb) ; 3,56 (d, 2H, Ha); 3,88(m, 1H),
ESI+ : [(M, H)+] = 206 m/z

### Exemple 24: Synthèse du 4-methyl-N-phenyl-1,2-dithiolane-4-carboxamide (Composé 29)

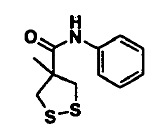

Même méthode que dans l'exemple 18 avec l'aniline.
On a obtenu 380 mg d'une huile jaune (Rdt 70%)
RMN 1H (DMSO-d6): δ ppm 1,51 (s,3H, Hc) ; 2,99 (d,2H, Hb) ; 3,75 (d, 2H, Ha), 7,08 (t, 1H, Ar), 7,3( t, 2H, Ar), 7.,60(d, 2H, Ar), 9,56(s, 1 h, NH)
ESI+ : [(M, H)+] = 240 m/z

### Exemple 25: Synthèse du N-butyl-4-methyl-1,2-dithiolane-4-carboxamide 1-oxide (composé 41)

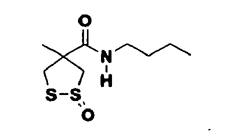

A 6 g de N- butyl -4-methyl-1,2-dithiolane-4-carboxamide (exemple 9 ; composé 11) dans 30 ml d'éthanol, 60 ml d'acétone et 15 ml d'acide acétique, ont été ajoutés 3 équivalents d'eau oxygénée. Le milieu réactionnel a été agité à 20°C pendant une nuit. Après concentration sous vide et purification sur colonne de silice (gradient Acétate d'éthyle/Heptane 60/40 à 80/20), on a obtienu le thiosulfinate sous forme d'un solide blanc mélange de 2 diastéréoisomères.

RMN 1H (DMSO-d6): δ ppm
Diastéréoisomère majoritaire : 8 (t, 1H), 4.21 (d, 1H), 3.94 (d, 1H), 3.55 (d, 1H), 3.36 (d, 1H), 3.07 (q, 2H), 1.49 (s, 3H)
ESI+ : [(M, H) +] = 236 m/z

### Exemple 26 : Synthèse du N-butyl-4-methyl-1,2-dithiolane-4-carboxamide 1-dioxide (composé 42)

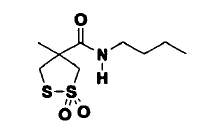

A 10.35 g de N- butyl -4-methyl-1,2-dithiolane-4-carboxamide (exemple 9 ; composé 11) dans 400 ml de méthanol, ont été ajoutés 58 g d'Oxone® (Aldrich CAS : 10058-23-8). Le milieu réactionnel a été agité à 20°C pendant une heure. Après filtration et concentration sous vide, le brut a été purifié sur colonne de silice (gradient Acétate d'éthyle/Heptane 60/40 à 80/20) pour conduire à 5 g du thiosulfonate sous forme d'un solide blanc.

RMN 1H (DMSO-d6): δ ppm 8 (t, 1H), 4.23 (d, 1H), 4.09 (d, 1H), 3.66 (d, 1H), 3.63 (d, 1H), 3.10 (q, 2H), 1.49 (s, 3H), 1.40 (m, 2H), 1.25 (m, 2H), 0.87 (t, 3H);
ESI+ : [(M, H) +] = 252 m/z

### Exemple 27 : Mise en évidence de l'activité sur la mélanogénèse constitutive

Un test biologique a mis en évidence l'activité dépigmentante des composés selon l'invention. L'effet modulateur sur la mélanogénèse constitutive des composés a été mesuré selon la méthode décrite dans le brevet FR-A-2734825, ainsi que dans l'article de Schmidt et al., Anal. Bichem., 235(2), 1996, pp.113-118. Ce test est réalisé sur coculture de kératinocytes et de mélanocytes.

Pour les composés testés, il a été déterminé l'activité inhibitrice sur la synthèse de la mélanine, en estimant le rapport de l'incorporation de thiouracile à l'incorporation de leucine, rapporté à 100% du témoin (le témoin correspond au test réalisé sans composé à tester).

### Les résultats sont rassemblés dans le tableau suivant :

| | **Cytotoxicité sur co-culture** | **Activité Maximale** |
|---|---|---|
| Composé 2 (exemple 5) | 100 µM | -76% |
| Arbutine | Non cytotoxique | -44% |
| Acide kojique | 100 µM | -30% |

Les résultats obtenus montrent que le composé 2 utilisé selon l'invention a une action dépigmentante plus importante que celle de l'arbutine et de l'acide kojique.

### Exemple 28

On prépare une crème blanchissante de soin du visage de type émulsion huile-dans-eau, comprenant (% en poids) :

| | |
|---|---|
| composé 2 (exemple 5) | 2% |
| stéarate de glycérol | 2% |
| polysorbate 60 (Tween 60 de ICI) | 1 % |
| acide stéarique | 1,4% |
| triéthanolamine | 0,7% |
| carbomer | 0,4% |
| fraction liquide du beurre de karité | 12% |
| perhydrosqualène | 12% |
| antioxydant | qs |
| parfum, conservateur | qs |
| eau | qsp 100% |

Une composition similaire est préparée avec le composé de l'exemple 9 (composé 11) ou de l'exemple 14 (composé 26).

### Exemple 29

On prépare un gel dépigmentant pour la peau comprenant (% en poids) :

| | |
|---|---|
| Composé 2 (exemple 5) | 2% |
| hydroxypropylcellulose (Klucel H de Hercules) | 1 % |
| antioxydant | qs |
| parfum, conservateur | qs |
| isopropanol | 40% |
| eau | qsp 100% |

Une composition similaire est préparée avec le composé de l'exemple 15 (composé 27).

## Revendications

1. Procédé cosmétique non thérapeutique de dépigmentation, d'éclaircissement et/ou de blanchiment des matières kératiniques, comprenant l'application d'une composition cosmétique comprenant, dans un milieu physiologiquement acceptable, au moins un composé de formule (I) : 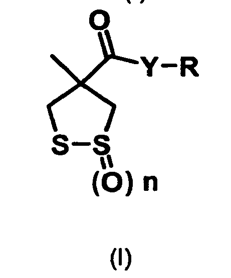 dans laquelle :
Y désigne O, NR₁ ou S
R₁ désigne un atome d'hydrogène ; un groupe hydrocarboné alkyle saturé linéaire en C₁-C₂₀ ou ramifié en C₃-C₂₀ ou insaturé en C₂-C₂₀ ; un groupe phényle éventuellement substitué par un ou plusieurs hydroxyles et/ou par un ou plusieurs radicaux alkoxy en C₁-C₈ ;
R désigne un atome d'hydrogène ; ou un groupe hydrocarboné alkyle saturé linéaire en C₁-G₂₀ ou ramifié en C₃-C₂₀ ou insaturé en C₂-C₂₀ ; ou un groupe phényle éventuellement substitué par un ou plusieurs hydroxyles et/ou par un ou plusieurs radicaux alkoxy en C₁-C₈ ; ou un groupe alkyle saturé en C₁-C₈ possédant un substituant phényle éventuellement substitué par un ou plusieurs hydroxyles et/ou par un ou plusieurs radicaux alcoxy en C₁-C₈ ;.
R possède éventuellement un ou plusieurs substituants choisis parmi OR₂, SR₂, NR₂R₃, COOR₂ dans lesquels :
R₂ désigne un atome d'hydrogène ou un groupe hydrocarboné saturé linéaire en C₁-C₅ ou ramifié en C₃-C₅ ou insaturé en C₂-C₅, ou un groupe phényle
R₃ désigne un atome d'hydrogène ; un groupe hydrocarboné saturé linéaire en C₁-C₅ ou ramifié en C₃-C₅ ou insaturé en C₂-C₅, ; un groupe phényle ; un groupe acétyle ; lorsque Y=NR1, R et R₁ peuvent former un cycle choisi parmi pyrrolidine, pyrroline, pipéridine, pipérazine, morpholine, thiomorpholine, azépine ;
n = 0 ou 1 ou 2,
ainsi que leurs sels, leurs chélates, leurs solvates et leurs isomères optiques.

2. Procédé selon la revendication 1, dans lequel :
Y désigne S, O, NR₁
R₁ désigne un atome d'hydrogène ; un groupe hydrocarboné alkyle saturé linéaire en C₁-C₁₀ ou ramifié en C₃-C₁₀ ;
R désigne un atome d'hydrogène ; un groupe hydrocarboné alkyle saturé linéaire en C₁-C₂₀ ou ramifié en C₃-C₂₀ ; un groupe phényle éventuellement substitué par un ou plusieurs hydroxyles et/ou par un ou plusieurs radicaux alcoxy en C₁-C₃ ; un groupe alkyle saturé en C₁-C₅ possédant un substituant phényle éventuellement substitué par un ou plusieurs hydroxyles et/ou par un ou plusieurs radicaux alcoxy en C₁-C₃ ; un groupe hydrocarboné alkyle linéaire en C1-C5 substitué par un ou plusieurs groupes identiques ou non choisis parmi OR₂, SR₂, NR₂R₃, COOR₂ dans lesquels:
R₂ désigne un atome d'hydrogène ou un groupe hydrocarboné saturé linéaire en C₁-C₅ ou ramifié en C₃-C₅ ou insaturé en C₂-C₅,
R₃ désigne un atome d'hydrogène ; un groupe hydrocarboné saturé linéaire en C₁-C₅ ou ramifié en C₃-C₅ ; un groupe phényle ; un groupe acétyle ;
lorsque Y=NR1, R et R₁ peuvent former un cycle pyrrolidine,
n = 0 ou 1 ou 2.

3. Procédé selon l'une des revendications précédentes, dans lequel :
Y désigne O ou NR₁ ;
R₁ désigne un atome d'hydrogène ; un groupe hydrocarboné alkyle saturé linéaire en C₁-C₁₀ ou ramifié en C₃-C₁₀ ;
R désigne un atome d'hydrogène ; un groupe hydrocarboné alkyle saturé linéaire en C₁-C₁₀ ou ramifié en C₃-C₁₀ ; un groupe phényle éventuellement substitué par un ou plusieurs hydroxyles et/ou par un ou plusieurs radicaux méthoxy ; un groupe hydrocarboné alkyle saturé en C₁-C₃ possédant un substituant phényle éventuellement substitué par un ou plusieurs hydroxyles et/ou par un ou plusieurs radicaux méthoxy ; un groupe hydrocarboné alkyle linéaire en C1-C4 substitué par un ou plusieurs groupes identiques ou non choisis parmi OR₂, SR₂, NR₂R₃, COOR₂ dans lesquels :
R₂ désigne un atome d'hydrogène ou un groupe hydrocarboné saturé linéaire en C₁-C₅ ou ramifié en C₃-C₅,
R₃ désigne un atome d'hydrogène ; un groupe hydrocarboné saturé linéaire en C₁-C₅ ou ramifié en C₃-C₅ ;
n = 0 ou 1 ou 2.

4. Procédé selon l'une des revendications précédentes, dans lequel
Y désigne NR₁ ;
R₁ désigne un atome d'hydrogène ; un groupe hydrocarboné alkyle saturé linéaire en C₁-C₄;
R désigne un atome d'hydrogène ; un groupe hydrocarboné alkyle saturé linéaire en C₁-C₁₀ ou ramifié en C₃-C₁₀ ; un groupe phényle ; un groupe alkyle saturé linéaire en C₁-C₄ substitué par un phényle substitué ou non par un ou plusieurs groupes identiques ou non choisis parmi OH, OMe ; un groupe hydrocarboné alkyle linéaire en C1-C4 substitué par un ou plusieurs groupes identiques ou non choisis parmi OH, NHAc, SR2, COOR2 avec R 2 étant un hydrogène ou un groupe alkyle linéaire en C1-C4 ;
n = 0 ou 1 ou 2 .

5. Procédé selon l'une des revendications précédentes, dans lequel
Y désigne NH
R désigne un atome d'hydrogène ; un groupe hydrocarboné alkyle saturé linéaire en C₁-C₁₀ ou ramifié en C₃-C₁₀ ; un groupe phényle ; un groupe alkyle saturé linéaire en C₁-C₄ substitué par un phényle substitué ou non par un ou plusieurs groupes identiques ou non choisis parmi OH, OMe ; un groupe hydrocarboné alkyle linéaire en C1-C4 substitué par un ou plusieurs groupes identiques ou non choisis parmi OH, NHAc, SR2, COOR2 avec R 2 étant un hydrogène ou un groupe alkyle linéaire en C1-C4 ;
n = 0 ou 1 ou 2.

6. Procédé selon la revendication 1, dans lequel le composé de formule (I) est choisi parmi les composés suivants :
- acide 4-méthyl-1,2-dithiolane-4-carboxylique
- 4-méthyl-1,2-dithiolane-4-carboxamide
- 4-méthyl-1,2-dithiolane-4-carboxylate de méthyle
- 4-méthyl-1,2-dithiolane-4-carboxylate d'éthyle
- 4-méthyl-1,2-dithiolane-4-carboxylate de propyle
- 4-méthyl-1,2-dithiolane-4-carboxylate de benzyle
- N-méthyl 4-méthyl-1,2-dithiolane-4-carboxamide
- acide {[(4-méthyl-1,2-dithiolan-4-yl)carbonyl]amino} acétique
- 4-méthyl-1,2-dithiolane-4-carboxylate d'octyle
- N-heptyl-4-méthyl-1,2-dithiolane-4-carboxamide
- N-butyl-4-méthyl-1,2-dithiolane-4-carboxamide
- 2-{[(4-méthyl-1,2-dithiolan-4-yl)carbonyl]amino}-4-(méthylsulfanyl)butanoate de méthyle
- S-[2-(acétylamino)éthyl] 4-méthyl-1,2-dithiolane-4-carbothioate
- N-(2-hydroxyéthyl)-4-méthyl-1,2-dithiolane-4-carboxamide
- N-(2,3-dihydroxypropyl)-4-méthyl-1,2-dithiolane-4-carboxamide
- N-(4-hydroxy-3-méthoxybenzyl)-4-méthyl-1,2-dithiolane-4-carboxamide
- N-[2-(4-hydroxy-3-méthoxyphenyl)éthyl]-4-méthyl-1,2-dithiolane-4-carboxamide
- N,N-diéthyl-4-méthyl-1,2-dithiolane-4-carboxamide
- 2-(acétylamino)-3-{[(4-méthyl-1,2-dithiolan-4-yl)carbonyl]sutfanyl}propanoate de méthyle
- S-(2-hydroxyéthyl) 4-méthyl-1,2-dithiolane-4-carbothioate
- {[(4-méthyl-1,2-dithiolan-4-yl)carbonyl]sulfanyl}acétate d'éthyle
- [(4-méthyl-1,2-dithiolan-4-yl)carbonyl]pyrrolidine
- Acide 4-méthyl-1,2-dithiolane-1-oxyde-4-carboxylique
- Acide 4-méthyl-1,2-dithiolane-1,1-dioxyde-4-carboxylique
- 4-méthyl-1,2-dithiolane-1-oxyde-4-carboxylate d'éthyle
- 4-méthyl-1,2-dithiolane-4-carboxamide 1-oxide
- 4-méthyl-1,2-dithiolane-4-carboxamide 1,1-dioxide
- 4-méthyl-N-(1-methylethyl)-1,2-dithiolane-4-carboxamide
- 4-méthyl-N-phenyl-1,2-dithiolane-4-carboxamide
- N-[2-(4-hydroxyphényl)éthyl]-4-méthyl-1,2-dithiolane-4-carboxamide
- N-propyl-4-méthyl-1,2-dithiolane-4-carboxamide
- N-pentyl-4-méthyl-1,2-dithiolane-4-carboxamide
- N-hexyl-4-méthyl-1,2-dithiolane-4-carboxamide
- N-octyl-4-méthyl-1,2-dithiolane-4-carboxamide
- N-propyl-4-méthyl-1,2-dithiolane-4-carboxamide
- 4-méthyl-1,2-dithiolane-4-carboxylate de butyle
- 4-méthyl-1,2-dithiolane-4-carboxylate d'isopropyle
- 4-méthyl-1,2-dithiolane-4-carboxylate de pentyle
- 4-méthyl-1,2-dithiolane-4-carboxylate d'hexyle
- 4-méthyl-1,2-dithiolane-4-carboxylate d'heptyle
- N-butyl-4-methyl-1,2-dithiolane-4-carboxamide 1-oxide
- N-butyl-4-methyl-1,2-dithiolane-4-carboxamide 1-dioxide

7. Procédé selon la revendication 1, dans lequel le composé de formule (I) est choisi parmi les composés suivants :
- 4-méthyl-1,2-dithiolane-4-carboxamide
- N-heptyl-4-méthyl-1,2-dithiolane-4-carboxamide
- N-butyl-4-méthyl-1,2-dithiolane-4-carboxamide
- acide 4-méthyl-1,2-dithiolane-1-oxyde-4-carboxylique
- acide 4-méthyl-1,2-dithiolane-1,1-dioxyde-4-carboxylique
- 4-méthyl-1,2-dithiolane-4-carboxamide 1-oxide
- 4-méthyl-1,2-dithiolane-4-carboxamide 1,1-dioxide
- N-butyl-4-methyl-1,2-dithiolane-4-carboxamide 1-oxide
- N-butyl-4-methyl-1,2-dithiolane-4-carboxamide 1-dioxide

8. Procédé selon l'une des revendications précédentes, dans lequel le composé de formule (I) est présent, seul ou en mélange, dans la composition, en une quantité comprise entre 0,01 et 10% en poids, de préférence entre 0,1 à 5% en poids, notamment de 0,5 à 3% en poids, par rapport au poids total de la composition.

9. Procédé selon l'une des revendications précédentes, dans lequel la composition comprend au moins un adjuvant choisi dans le groupe formé par : l'eau; les solvants organiques, notamment les alcools en C1-C6 et les esters d'acide carboxylique en C2-C10; les huiles carbonées et/ou siliconées, d'origine minérale, animale et/ou végétale; les cires, les pigments, les charges, les colorants, les tensioactifs, les émulsionnants, les co-émulsionnants; les actifs cosmétiques ou dermatologiques, les polymères, les gélifiants hydrophiles ou lipophiles, les épaississants, les conservateurs, les parfums, les bactéricides, les céramides, les absorbeurs d'odeur, les antioxydants.

10. Procédé selon l'une des revendications précédentes, dans lequel la composition comprend au moins un actif choisi parmi: les agents desquamants; les agents apaisants, les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaire; les agents agissant sur la microcirculation; les agents agissant sur le métabolisme énergétique des cellules; et leurs mélanges.

11. Procédé selon l'une des revendications précédentes, pour dépigmenter, éclaircir et/ou blanchir la peau.

12. Utilisation cosmétique non thérapeutique d'un composé de formule (I) tel que défini selon l'une quelconque des revendications 1 à 7, comme agent blanchissant, éclaircissant et/ou dépigmentant des matières kératiniques.

## Patentansprüche

1. Nichttherapeutisches kosmetisches Verfahren zur Depigmentierung, Aufhellung und/oder Bleichung von Keratinmaterialien, umfassend das Aufbringen einer kosmetischen Zusammensetzung, die in einem physiologisch unbedenklichen Medium mindestens eine Verbindung der Formel (I): 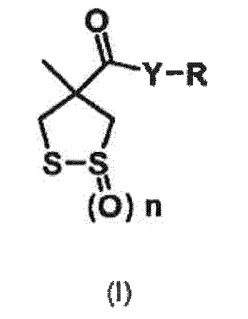 worin:
Y für O, NR₁ oder S steht;
R₁ für ein Wasserstoffatom; eine gesättigte lineare C₁-C₂₀- oder verzweigte C₃-C₂₀- oder ungesättigte C₂-C₂₀-Alkylkohlenwasserstoffgruppe oder eine Phenylgruppe, die gegebenenfalls durch eine oder mehrere Hydroxylgruppen und/oder durch einen oder mehrere C₁-C₈-Alkoxyreste substituiert ist, steht;
R für ein Wasserstoffatom oder eine gesättigte lineare C₁-C₂₀- oder verzweigte C₃-C₂₀- oder ungesättigte C₂-C₂₀-Alkylkohlenwasserstoffgruppe oder eine Phenylgruppe, die gegebenenfalls durch eine oder mehrere Hydroxylgruppen und/oder durch einen oder mehrere C₁-C₈-Alkoxyreste substituiert ist, oder eine gesättigte C₁-C₈-Alkylgruppe mit einem Phenylsubstituenten, der gegebenenfalls durch eine oder mehrere Hydroxylgruppen und/oder durch einen oder mehrere C₁-C₈-Alkoxyreste substituiert ist, steht;
R gegebenenfalls einen oder mehrere Substituenten, die aus OR₂, SR₂, NR₂R₃ und COOR₂ ausgewählt sind, aufweist, wobei:
R₂ für ein Wasserstoffatom oder eine gesättigte lineare C₁-C₅- oder verzweigte C₃-C₅- oder ungesättigte C₂-C₅-Kohlenwasserstoffgruppe oder eine Phenylgruppe steht;
R₃ für ein Wasserstoffatom; eine gesättigte lineare C₁-C₅- oder verzweigte C₃-C₅- oder ungesättigte C₂-C₅-Kohlenwasserstoffgruppe; eine Phenylgruppe oder eine Acetylgruppe steht;
R und R₁ dann, wenn Y = NR₁, einen Ring, der unter Pyrrolidin, Pyrrolin, Piperidin, Piperazin, Morpholin, Thiomorpholin und Azepin ausgewählt ist, bilden können;
n = 0 oder 1 oder 2,
sowie Salze davon, Chelate davon, Solvate davon und optische Isomere davon
umfasst.

2. Verfahren nach Anspruch 1, bei dem:
Y für S, O oder NR₁ steht;
R₁ für ein Wasserstoffatom oder eine gesättigte lineare C₁-C₁₀- oder verzweigte C₃-C₁₀-Alkylkohlen-wasserstoffgruppe steht;
R für ein Wasserstoffatom; eine gesättigte lineare C₁-C₂₀- oder verzweigte C₃-C₂₀-Alkylkohlenwasser-stoffgruppe; eine Phenylgruppe, die gegebenenfalls durch eine oder mehrere Hydroxylgruppen und/oder durch einen oder mehrere C₁-C₃-Alkoxyreste substituiert ist; eine gesättigte C₁-C₅-Alkylgruppe mit einem Phenylsubstituenten, der gegebenenfalls durch eine oder mehrere Hydroxylgruppen und/oder durch einen oder mehrere C₁-C₃-Alkoxyreste substituiert ist; oder eine lineare C₁-C₅-Alkyl-kohlenwasserstoffgruppe, die durch eine oder
mehrere gleiche oder verschiedene Gruppen, die aus OR₂, SR₂, NR₂R₃ und COOR₂ ausgewählt sind,
substituiert ist, steht, wobei:
R₂ für ein Wasserstoffatom oder eine gesättigte lineare C₁-C₅- oder verzweigte C₃-C₅- oder ungesättigte C₂-C₅-Kohlenwasserstoffgruppe steht;
R₃ für ein Wasserstoffatom; eine gesättigte lineare C₁-C₅- oder verzweigte C₃-C₅-Kohlenwasserstoffgruppe; eine Phenylgruppe oder eine Acetylgruppe steht;
R und R₁ dann, wenn Y = NR₁, einen Pyrrolidinring bilden können;
n = 0 oder 1 oder 2.

3. Verfahren nach einem der vorhergehenden Ansprüche, bei dem:
Y für O oder NR₁ steht;
R₁ für ein Wasserstoffatom oder eine gesättigte lineare C₁-C₁₀- oder verzweigte C₃-C₁₀-Alkylkohlen-wasserstoffgruppe steht;
R für ein Wasserstoffatom; eine gesättigte lineare C₁-C₁₀- oder verzweigte C₃-C₁₀-Alkylkohlenwasser-stoffgruppe; eine Phenylgruppe, die gegebenenfalls durch eine oder mehrere Hydroxylgruppen und/oder durch einen oder mehrere Methoxyreste substituiert ist; eine gesättigte C₁-C₃-Alkyl-kohlenwasserstoffgruppe mit einem Phenylsubstituenten, der gegebenenfalls durch eine oder mehrere Hydroxylgruppen und/oder durch einen oder mehrere Methoxyreste substituiert ist; oder eine lineare C₁-C₄-Alkylkohlenwasserstoffgruppe, die durch eine oder mehrere gleiche oder verschiedene Gruppen, die aus OR₂, SR₂, NR₂R₃ und COOR₂ ausgewählt sind, substituiert ist, steht, wobei:
R₂ für ein Wasserstoffatom oder eine gesättigte lineare C₁-C₅- oder verzweigte C₃-C₅-Kohlenwasserstoffgruppe steht;
R₃ für ein Wasserstoffatom oder eine gesättigte lineare C₁-C₅- oder verzweigte C₃-C₅-Kohlenwasser-stoffgruppe steht;
n = 0 oder 1 oder 2.

4. Verfahren nach einem der vorhergehenden Ansprüche, bei dem:
Y für NR₁ steht;
R₁ für ein Wasserstoffatom oder eine gesättigte lineare C₁-C₄-Alkylkohlenwasserstoffgruppe steht;
R für ein Wasserstoffatom; eine gesättigte lineare C₁-C₁₀- oder verzweigte C₃-C₁₀-Alkylkohlenwasserstoffgruppe; eine Phenylgruppe; eine gesättigte lineare C₁-C₄-Alkylgruppe, die durch ein Phenyl,
das gegebenenfalls durch eine oder mehrere gleiche oder verschiedene Gruppen, die unter OH und OMe ausgewählt sind, substituiert ist, substituiert ist; oder eine lineare C₁-C₄-Alkylkohlenwasserstoffgruppe, die durch eine oder mehrere gleiche oder verschiedene Gruppen, die aus OH, NHAc, SR₂ und COOR₂ ausgewählt sind, substituiert ist, steht,
wobei R₂ für ein Wasserstoffatom oder eine lineare C₁-C₄-Alkylgruppe steht;
n = 0 oder 1 oder 2.

5. Verfahren nach einem der vorhergehenden Ansprüche, bei dem
Y für NH steht;
R für ein Wasserstoffatom; eine gesättigte lineare C₁-C₁₀- oder verzweigte C₃-C₁₀-Alkylkohlenwasserstoffgruppe; eine Phenylgruppe; eine gesättigte lineare C₁-C₄-Alkylgruppe, die durch ein Phenyl, das gegebenenfalls durch eine oder mehrere gleiche oder verschiedene Gruppen, die unter OH und OMe ausgewählt sind, substituiert ist, substituiert ist; oder eine lineare C₁-C₄-Alkylkohlenwasserstoffgruppe, die durch eine oder mehrere gleiche oder verschiedene Gruppen, die aus OH, NHAc, SR₂ und COOR₂ ausgewählt sind, substituiert ist, steht, wobei R₂ für ein Wasserstoffatom oder eine lineare C₁-C₄-Alkylgruppe steht;
n = 0 oder 1 oder 2.

6. Verfahren nach Anspruch 1, bei dem die Verbindung der Formel (I) unter den folgenden Verbindungen ausgewählt ist:
- 4-Methyl-1,2-dithiolan-4-carbonsäure
- 4-Methyl-1,2-dithiolan-4-carboxamid
- 4-Methyl-1,2-dithiolan-4-carbonsäuremethylester
- 4-Methyl-1,2-dithiolan-4-carbonsäureethylester
- 4-Methyl-1,2-dithiolan-4-carbonsäurepropylester
- 4-Methyl-1,2-dithiolan-4-carbonsäurebenzylester
- N-Methyl-4-methyl-1,2-dithiolan-4-carboxamid
- {[(4-Methyl-1,2-dithiolan-4-yl)carbonyl]amino}-essigsäure
- 4-Methyl-1,2-dithiolan-4-carbonsäureoctylester
- N-Heptyl-4-methyl-1,2-dithiolan-4-carboxamid
- N-Butyl-4-methyl-1,2-dithiolan-4-carboxamid
- 2-{[(4-Methyl-1,2-dithiolan-4-yl)carbonyl]-amino}-4-(methylsulfanyl)butansäuremethylester
- 4-Methyl-1,2-dithiolan-4-thiocarbonsäure-S-[2-(acetylamino)ethyl]ester
- N-(2-Hydroxyethyl)-4-methyl-1,2-dithiolan-4-carboxamid
- N-(2,3-Dihydroxypropyl)-4-methyl-1,2-dithiolan-4-carboxamid
- N-(4-Hydroxy-3-methoxybenzyl)-4-methyl-1,2-dithiolan-4-carboxamid
- N-[2-(4-Hydroxy-3-methoxyphenyl)ethyl]-4-methyl-1,2-dithiolan-4-carboxamid
- N,N-Diethyl-4-methyl-1,2-dithiolan-4-carboxamid
- 2-(Acetylamino)-3-{[(4-methyl-1,2-dithiolan-4-yl)carbonyl]sulfanyl}propansäuremethylester
- 4-Methyl-1,2-dithiolan-4-thiocarbonsäure-S-(2-hydroxyethyl)ester
- {[(4-Methyl-1,2-dithiolan-4-yl)carbonyl]-sulfanyl}essigsäureethylester
- [(4-Methyl-1,2-dithiolan-4-yl)carbonyl]-pyrrolidin
- 4-Methyl-1,2-dithiolan-1-oxo-4-carbonsäure
- 4-Methyl-1,2-dithiolan-1,1-dioxo-4-carbonsäure
- 4-Methyl-1,2-dithiolan-1-oxo-4-carbonsäure-ethylester
- 4-Methyl-1,2-dithiolan-4-carboxamid-1-oxid
- 4-Methyl-1,2-dithiolan-4-carboxamid-1,1-dioxid
- 4-Methyl-N-(1-methylethyl)-1,2-dithiolan-4-carboxamid
- 4-Methyl-N-phenyl-1,2-dithiolan-4-carboxamid
- N-[2-(4-Hydroxyphenyl)ethyl]-4-methyl-1,2-dithiolan-4-carboxamid
- N-Propyl-4-methyl-1,2-dithiolan-4-carboxamid
- N-Pentyl-4-methyl-1,2-dithiolan-4-carboxamid
- N-Hexyl-4-methyl-1,2-dithiolan-4-carboxamid
- N-Octyl-4-methyl-1,2-dithiolan-4-carboxamid
- N-Propyl-4-methyl-1,2-dithiolan-4-carboxamid
- 4-Methyl-1,2-dithiolan-4-carbonsäurebutylester
- 4-Methyl-1,2-dithiolan-4-carbonsäureisopropyl-ester
- 4-Methyl-1,2-dithiolan-4-carbonsäurepentylester
- 4-Methyl-1,2-dithiolan-4-carbonsäurehexylester
- 4-Methyl-1,2-dithiolan-4-carbonsäureheptylester
- N-Butyl-4-methyl-1,2-dithiolan-4-carboxamid-1-oxid
- N-Butyl-4-methyl-1,2-dithiolan-4-carboxamid-1,1-dioxid.

7. Verfahren nach Anspruch 1, bei dem die Verbindung der Formel (I) unter den folgenden Verbindungen ausgewählt ist:
- 4-Methyl-1,2-dithiolan-4-carboxamid
- N-Heptyl-4-methyl-1,2-dithiolan-4-carboxamid
- N-Butyl-4-methyl-1,2-dithiolan-4-carboxamid
- 4-Methyl-1,2-dithiolan-1-oxo-4-carbonsäure
- 4-Methyl-1,2-dithiolan-1,1-dioxo-4-carbonsäure
- 4-Methyl-1,2-dithiolan-4-carboxamid-1-oxid
- 4-Methyl-1,2-dithiolan-4-carboxamid-1,1-dioxid
- N-Butyl-4-methyl-1,2-dithiolan-4-carboxamid-1-oxid
- N-Butyl-4-methyl-1,2-dithiolan-4-carboxamid-1-dioxid.

8. Verfahren nach einem der vorhergehenden Ansprüche, bei dem die Verbindung der Formel (I) alleine oder im Gemisch in der Zusammensetzung in einer Menge zwischen 0,01 und 10 Gew.-%, vorzugsweise zwischen 0,1 und 5 Gew.-%, insbesondere 0,5 bis 3 Gew.-%, bezogen auf das Gesamtgewicht der Zusammensetzung, vorliegt.

9. Verfahren nach einem der vorhergehenden Ansprüche, bei dem die Zusammensetzung mindestens einen Hilfsstoff aus der Gruppe bestehend aus Wasser; organischen Lösungsmitteln, insbesondere C₁-C₆-Alkoholen und C₂-C₁₀-Carbonsäureestern; auf Kohlenstoff basierenden Ölen und/oder Silikonölen mineralischer, tierischer und/oder pflanzlicher Herkunft; Wachsen, Pigmenten, Füllstoffen, Farbmitteln, Tensiden, Emulgatoren, Coemulgatoren; kosmetischen oder dermatologischen Wirkstoffen, Polymeren, hydrophilen oder lipophilen Geliermitteln, Verdickungsmitteln, Konservierungsmitteln, Duftstoffen, Bakteriziden, Ceramiden, Geruchsabsorbern und Antioxidantien umfasst.

10. Verfahren nach einem der vorhergehenden Ansprüche, bei dem die Zusammensetzung mindestens einen Wirkstoff, der aus Entschuppungsmitteln; beruhigend wirkenden Mitteln, Feuchtigkeitsmitteln; depigmentierenden oder propigmentierenden Mitteln; Antiglykierungsmitteln; NO-Synthase-Inhibitoren; Mitteln zur Stimulierung der Synthese von dermalen oder epidermalen Makromolekülen und/oder zur Verhinderung ihrer Degradation; Mitteln zur Stimulierung der Fibroblasten- und/oder Keratinozytenproliferation oder zur Stimulierung der Keratinozytendifferenzierung; Muskelrelaxantien und/oder Hautdekontraktionsmitteln; Straffungsmitteln; Mitteln gegen Schadstoffe und/oder Radikale; Mitteln, die auf die Mikrozirkulation wirken; Mitteln, die auf den Energiestoffwechsel von Zellen wirken; und Mischungen davon ausgewählt ist, umfasst.

11. Verfahren nach einem der vorhergehenden Ansprüche zur Depigmentierung, Aufhellung und/oder Bleichung der Haut.

12. Nichttherapeutische kosmetische Verwendung einer Verbindung der Formel (I) gemäß einem der Ansprüche 1 bis 7 als Mittel zur Bleichung, Aufhellung und/oder Depigmentierung von Keratinmaterialien.

## Claims

1. Non-therapeutic cosmetic process for depigmenting, lightening and/or bleaching keratin materials, comprising the application of a cosmetic composition comprising, in a physiologically acceptable medium, at least one compound of formula (I): 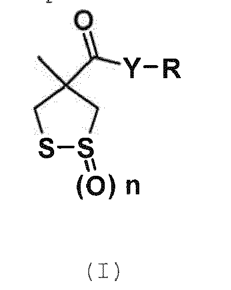 in which:
Y denotes O, NR₁ or S
R₁ denotes a hydrogen atom; a saturated linear C₁-C₂₀ or
branched C₃-C₂₀ or unsaturated C₂-C₂₀ alkyl hydrocarbon-based group; a phenyl group optionally substituted with one or more hydroxyls and/or with one or more C₁-C₈ alkoxy radicals;
R denotes a hydrogen atom; or a saturated linear C₁-C₂₀ or branched C₃-C₂₀ or unsaturated C₂-C₂₀ alkyl hydrocarbon-based group; or a phenyl group optionally substituted with one or more hydroxyls and/or with one or more C₁-C₈ alkoxy radicals; or a saturated C₁-C₈ alkyl group containing a phenyl substituent optionally substituted with one or more hydroxyls and/or with one or more C₁-C₈ alkoxy radicals;
R optionally bears one or more substituents chosen from OR₂, SR₂, NR₂R₃, COOR₂ in which:
R₂ denotes a hydrogen atom or a saturated linear C₁-C₅ or branched C₃-C₅ or unsaturated C₂-C₅ hydrocarbon-based group, or a phenyl group
R₃ denotes a hydrogen atom; a saturated linear C₁-C₅ or branched C₃-C₅ or unsaturated C₂-C₅ hydrocarbon-based group; a phenyl group; an acetyl group;
when Y = NR₁, R and R₁ may form a ring chosen from pyrrolidine, pyrroline, piperidine, piperazine, morpholine, thiomorpholine and azepine;
n = 0 or 1 or 2,
and also the salts thereof, chelates thereof, solvates thereof and optical isomers thereof.

2. Process according to Claim 1, in which:
Y denotes S, O, NR₁
R₁ denotes a hydrogen atom; a saturated linear C₁-C₁₀ or branched C₃-C₁₀ alkyl hydrocarbon-based group;
R denotes a hydrogen atom; a saturated linear C₁-C₂₀ or branched C₃-C₂₀ alkyl hydrocarbon-based group; a phenyl group optionally substituted with one or more hydroxyls and/or with one or more C₁-C₃ alkoxy radicals; a saturated C₁-C₅ alkyl group containing a phenyl substituent optionally substituted with one or more hydroxyls and/or with one or more C₁-C₃ alkoxy radicals;
a linear C₁-C₅ alkyl hydrocarbon-based group substituted with one or more identical or different groups chosen from OR₂, SR₂, NR₂R₃, COOR₂ in which:
R₂ denotes a hydrogen atom or a saturated linear C₁-C₅ or branched C₃-C₅ or unsaturated C₂-C₅ hydrocarbon-based group,
R₃ denotes a hydrogen atom; a saturated linear C₁-C₅ or branched C₃-C₅ hydrocarbon-based group; a phenyl group;
an acetyl group;
when Y = NR₁, R and R₁ may form a pyrrolidine ring,
n = 0 or 1 or 2.

3. Process according to one of the preceding claims, in which:
Y denotes O or NR₁;
R₁ denotes a hydrogen atom; a saturated linear C₁-C₁₀ or
branched C₃-C₁₀ alkyl hydrocarbon-based group;
R denotes a hydrogen atom; a saturated linear C₁-C₁₀ or
branched C₃-C₁₀ alkyl hydrocarbon-based group; a phenyl group optionally substituted with one or more hydroxyls and/or with one or more methoxy radicals; a saturated C₁-C₃ alkyl hydrocarbon-based group containing a phenyl substituent optionally substituted with one or more hydroxyls and/or with one or more methoxy radicals; a linear C₁-C₄ alkyl hydrocarbon-based group substituted with one or more identical or different groups chosen from OR₂, SR₂, NR₂R₃, COOR₂ in which:
R₂ denotes a hydrogen atom or a saturated linear C₁-C₅ or branched C₃-C₅ hydrocarbon-based group,
R₃ denotes a hydrogen atom; a saturated linear C₁-C₅ or branched C₃-C₅ hydrocarbon-based group;
n = 0 or 1 or 2.

4. Process according to one of the preceding claims, in which
Y denotes NR₁;
R₁ denotes a hydrogen atom; a saturated linear C₁-C₄ alkyl hydrocarbon-based group;
R denotes a hydrogen atom; a saturated linear C₁-C₁₀ or branched C₃-C₁₀ alkyl hydrocarbon-based group; a phenyl group; a saturated linear C₁-C₄ alkyl group substituted with a phenyl optionally substituted with one or more identical or different groups chosen from OH, OMe; a linear C₁-C₄ alkyl hydrocarbon-based group substituted with one or more identical or different groups chosen from OH, NHAc, SR₂, COOR₂ with R₂ being a hydrogen or a linear C₁-C₄ alkyl group;
n = 0 or 1 or 2.

5. Process according to one of the preceding claims, in which
Y denotes NH
R denotes a hydrogen atom; a saturated linear C₁-C₁₀ or branched C₃-C₁₀ alkyl hydrocarbon-based group; a phenyl group; a saturated linear C₁-C₄ alkyl group substituted with a phenyl optionally substituted with one or more identical or different groups chosen from OH, OMe; a linear C₁-C₄ alkyl hydrocarbon-based group substituted with one or more identical or different groups chosen from OH, NHAc, SR₂, COOR₂ with R₂ being a hydrogen or a linear C₁-C₄ alkyl group;
n = 0 or 1 or 2.

6. Process according to Claim 1, in which the compound of formula (I) is chosen from the following compounds:
- 4-methyl-1,2-dithiolane-4-carboxylic acid
- 4-methyl-1,2-dithiolane-4-carboxamide
- methyl 4-methyl-1,2-dithiolane-4-carboxylate
- ethyl 4-methyl-1,2-dithiolane-4-carboxylate
- propyl 4-methyl-1,2-dithiolane-4-carboxylate
- benzyl 4-methyl-1,2-dithiolane-4-carboxylate
- N-methyl-4-methyl-1,2-dithiolane-4-carboxamide
- {[(4-methyl-1,2-dithiolan-4-yl)carbonyl]amino}acetic acid
- octyl 4-methyl-1,2-dithiolane-4-carboxylate
- N-heptyl-4-methyl-1,2-dithiolane-4-carboxamide
- N-butyl-4-methyl-1,2-dithiolane-4-carboxamide
- methyl 2-{[(4-methyl-1,2-dithiolan-4-yl)carbonyl]amino}-4-(methylsulfanyl)butanoate
- S-[2-(acetylamino)ethyl] 4-methyl-1,2-dithiolane-4-carbothioate
- N-(2-hydroxyethyl)-4-methyl-1,2-dithiolane-4-carboxamide
- N-(2,3-dihydroxypropyl)-4-methyl-1,2-dithiolane-4-carboxamide
- N-(4-hydroxy-3-methoxybenzyl)-4-methyl-1,2-dithiolane-4-carboxamide
- N-[2-(4-hydroxy-3-methoxyphenyl)ethyl]-4-methyl-1,2-dithiolane-4-carboxamide
- N,N-diethyl-4-methyl-1,2-dithiolane-4-carboxamide
- methyl 2-(acetylamino)-3-{[(4-methyl-1,2-dithiolan-4-yl)carbonyl]sulfanyl}propanoate
- S-(2-hydroxyethyl) 4-methyl-1,2-dithiolane-4-carbothioate
- ethyl {[(4-methyl-1,2-dithiolan-4-yl)carbonyl]sulfanyl}acetate
- [(4-methyl-1,2-dithiolan-4-yl)carbonyl]pyrrolidine
- 4-methyl-1,2-dithiolane-1-oxo-4-carboxylic acid
- 4-methyl-1,2-dithiolane-1,1-dioxo-4-carboxylic acid
- ethyl 4-methyl-1,2-dithiolane-1-oxo-4-carboxylate
- 4-methyl-1,2-dithiolane-4-carboxamide 1-oxide
- 4-methyl-1,2-dithiolane-4-carboxamide 1,1-dioxide
- 4-methyl-N-(1-methylethyl)-1,2-dithiolane-4-carboxamide
- 4-methyl-N-phenyl-1,2-dithiolane-4-carboxamide
- N-[2-(4-hydroxyphenyl)ethyl]-4-methyl-1,2-dithiolane-4-carboxamide
- N-propyl-4-methyl-1,2-dithiolane-4-carboxamide
- N-pentyl-4-methyl-1,2-dithiolane-4-carboxamide
- N-hexyl-4-methyl-1,2-dithiolane-4-carboxamide
- N-octyl-4-methyl-1,2-dithiolane-4-carboxamide
- N-propyl-4-methyl-1,2-dithiolane-4-carboxamide
- butyl 4-methyl-1,2-dithiolane-4-carboxylate
- isopropyl 4-methyl-1,2-dithiolane-4-carboxylate
- pentyl 4-methyl-1,2-dithiolane-4-carboxylate
- hexyl 4-methyl-1,2-dithiolane-4-carboxylate
- heptyl 4-methyl-1,2-dithiolane-4-carboxylate
- N-butyl-4-methyl-1,2-dithiolane-4-carboxamide 1-oxide
- N-butyl-4-methyl-1,2-dithiolane-4-carboxamide 1-dioxide.

7. Process according to Claim 1, in which the compound of formula (I) is chosen from the following compounds:
- 4-methyl-1,2-dithiolane-4-carboxamide
- N-heptyl-4-methyl-1,2-dithiolane-4-carboxamide
- N-butyl-4-methyl-1,2-dithiolane-4-carboxamide
- 4-methyl-1,2-dithiolane-1-oxo-4-carboxylic acid
- 4-methyl-1,2-dithiolane-1,1-dioxo-4-carboxylic acid
- 4-methyl-1,2-dithiolane-4-carboxamide 1-oxide
- 4-methyl-1,2-dithiolane-4-carboxamide 1,1-dioxide
- N-butyl-4-methyl-1,2-dithiolane-4-carboxamide 1-oxide
- N-butyl-4-methyl-1,2-dithiolane-4-carboxamide 1-dioxide.

8. Process according to one of the preceding claims, in which the compound of formula (I) is present, alone or as a mixture, in the composition in an amount of between 0.01% and 10% by weight, preferably between 0.1% and 5% by weight and especially from 0.5% to 3% by weight relative to the total weight of the composition.

9. Process according to one of the preceding claims, in which the composition comprises at least one adjuvant chosen from the group formed by: water; organic solvents, especially C₁-C₆ alcohols and C₂-C₁₀ carboxylic acid esters; carbon-based and/or silicone oils, of mineral, animal and/or plant origin; waxes, pigments, fillers, colorants, surfactants, emulsifiers, co-emulsifiers; cosmetic or dermatological active agents, polymers, hydrophilic or lipophilic gelling agents, thickeners, preserving agents, fragrances, bactericides, ceramides, odour absorbers, antioxidants.

10. Process according to one of the preceding claims, in which the composition comprises at least one active agent chosen from: desquamating agents; calmatives, moisturizers; depigmenting or propigmenting agents; anti-glycation agents; NO-synthase inhibitors; agents for stimulating the synthesis of dermal or epidermal macromolecules and/or for preventing their degradation; agents for stimulating fibroblast and/or keratinocyte proliferation or for stimulating keratinocyte differentiation; muscle relaxants and/or dermo-decontracting agents; tensioning agents; anti-pollution agents and/or free-radical scavengers; agents acting on the capillary circulation; agents acting on the energy metabolism of cells; and mixtures thereof.

11. Process according to one of the preceding claims, for depigmenting, lightening and/or bleaching the skin.

12. Non therapeutic cosmetic use of a compound of formula (I) as defined according to any one of Claims 1 to 7, as an agent for bleaching, lightening and/or depigmenting keratin materials.
